# EUROPEAN PATENT SPECIFICATION

(11) **EP 4 272 634 B1**
(45) Date of publication and mention of the grant of the patent: **27.08.2025**
(21) Application number: 21914283.3
(22) Date of filing: 27.12.2021
(51) Int. Cl.: A61B 5/024, A61B 5/02, A61B 5/00

(54) **PARAMETER DETERMINATION METHOD, PARAMETER DETERMINATION APPARATUS, STORAGE MEDIUM AND ELECTRONIC DEVICE**
PARAMETERBESTIMMUNGSVERFAHREN, PARAMETERBESTIMMUNGSVORRICHTUNG, SPEICHERMEDIUM UND ELEKTRONISCHE VORRICHTUNG
PROCÉDÉ DE DÉTERMINATION DE PARAMÈTRES, APPAREIL DE DÉTERMINATION DE PARAMÈTRES, SUPPORT D'ENREGISTREMENT ET DISPOSITIF ÉLECTRONIQUE

(30) Priority: 30.12.2020 CN 202011624658
(43) Date of publication of application: 08.11.2023
(73) Proprietor: Wang, Li, Shanghai 201315 (CN)
(72) Inventor: Wang, Li, Shanghai 201315 (CN)
(74) Representative: Murgitroyd & Company
(86) International application number: PCT/CN2021/141724
(87) International publication number: WO 2022/143547

(56) References cited:
- CN-A- 103 209 637
- CN-A- 103 717 125
- CN-A- 105 943 014
- CN-A- 106 551 691
- CN-A- 107 480 414
- CN-A- 108 135 534
- US-A1- 2016 256 060
- US-A1- 2018 116 607
- US-A1- 2020 214 644
- US-A1- 2020 297 955
- US-B1- 10 582 862
- MIRJANA M PLATISA ET AL: "Reflection of heart rate regulation on linear and nonlinear heart rate variability measures; Relationships between HRV measures", PHYSIOLOGICAL MEASUREMENT, INSTITUTE OF PHYSICS PUBLISHING, BRISTOL, GB, vol. 27, no. 2, 1 February 2006 (2006-02-01), pages 145 - 154, XP020105729, ISSN: 0967-3334, DOI: 10.1088/0967-3334/27/2/005
- VANDEPUT S ET AL: "Nonlinear heart rate dynamics: Circadian profile and influence of age and gender", MEDICAL ENGINEERING & PHYSICS, BUTTERWORTH-HEINEMANN, GB, vol. 34, no. 1, 11 July 2011 (2011-07-11), pages 108 - 117, XP028438561, ISSN: 1350-4533, [retrieved on 20110714], DOI: 10.1016/J.MEDENGPHY.2011.07.004
- ADAMOPOULOS S ET AL: "Comparison of different methods for assessing sympathovagal balance in chronic congestive heart failure secondary to coronary artery disease", AMERICAN JOURNAL OF CARDIOLOGY, CAHNERS PUBLISHING CO., NEWTON, MA, US, vol. 70, no. 20, 15 December 1992 (1992-12-15), pages 1576 - 1582, XP023209285, ISSN: 0002-9149, [retrieved on 19921215], DOI: 10.1016/0002-9149(92)90460-G

## Description

### TECHNICAL FIELD

The present disclosure relates to the field of medical data processing technologies, and in particular, to a parameter determination method, a parameter determination apparatus, a non-transitory computer-readable storage medium, and an electronic device.

### BACKGROUND

As is well known, neurohormones driven by sympathetic and parasympathetic nerves are highly susceptible to various factors such as circadian rhythm, course of disease, sleep status, and medication, thereby being difficult to be accurately measured.

Therefore, a parameter determination method is urgently needed to determine a parameter accurately characterizing a level and/or a state of neurohormone.

US10582862B1 discloses a sensor device for detecting basal heart rate. A moving average method and a moving standard deviation algorithm are used to calculate the base heart rate. Further, the values of the basal heart rate and the values of the heart rate variability parameters for a 24-hour interval are input into a machine learning module, a feature extraction module, and a prediction model module, and daytime hours and bedtime hours are distinguished for predicting cardiovascular health risk factors.

XP20105729A discloses a linear and nonlinear measurement of heart rate variability and their relationship to RR interval length. The respective mean RR values during wakefulness and sleep were selected and the mean value of heart rate variability was calculated from the respective mean RR values. The relationship between heart rate variability and different parameters is mainly discussed.

XP28438561A discloses a method of measuring heart rate variability, associated with a variety of parameters associated with heart rate variability.

XP23209285A discloses different methods for assessing sympathovagal Balance in chronic congestive heart failure secondary to coronary artery disease.

### SUMMARY

To solve technical problems mentioned above, the present disclosure is proposed. A parameter determination method, a parameter determination apparatus, a non-transitory computer-readable storage medium, and an electronic device are provided by embodiments of the present disclosure.

According to an aspect, an embodiment of the present disclosure provides a parameter determination method, including: determining, based on a first time interval, a first parameter set corresponding to a subject to be tested, where the first time interval includes a nighttime interval, the first parameter set includes a first heart-rate characterization parameter and a first heart-rate variability characterization parameter, the first heart-rate characterization parameter includes a first mean heart-rate parameter and a first static heart-rate parameter, and the first heart-rate variability characterization parameter includes a first static heart-rate variability parameter; and determining, based on a second time interval, a second parameter set corresponding to the subject to be tested, where the second time interval includes a daytime interval, the second parameter set includes a second heart-rate characterization parameter and a second heart-rate variability characterization parameter, the second heart-rate characterization parameter includes a second mean heart-rate parameter and a second static heart-rate parameter, and the second heart-rate variability characterization parameter includes a second static heart-rate variability parameter; characterized in that the first time interval includes a plurality of first time periods, the determining, based on a first time interval, a first parameter set corresponding to a subject to be tested includes: determining , based on the plurality of first time periods, first mean heart-rate parameters respectively corresponding to the plurality of first time periods; determining, based on the first mean heart-rate parameters respectively corresponding to the plurality of first time periods, the first static heart-rate parameter, where the first static heart-rate parameter includes a sleep rest heart rate parameter; determining a first time period corresponding to the first static heart-rate parameter as a first static time period; and determining a heart-rate variability parameter corresponding to the first static time period as the first static heart-rate variability parameter, where the first static heart-rate variability parameter includes a sleep rest heart rate variability parameter; the second time interval includes a plurality of second time periods, and the determining, based on a second time interval, a second parameter set corresponding to the subject to be tested includes: determining, based on the plurality of second time periods, second mean heart-rate parameters respectively corresponding to the plurality of second time periods; determining, based on the second mean heart-rate parameters respectively corresponding to the plurality of second time periods, the second static heart-rate parameter, where the second static heart-rate parameter includes a daytime-rest heart-rate parameter; determining a second time period corresponding to the second static heart-rate parameter as a third static time period; and determining a heart-rate variability parameter corresponding to the third static time period as the second static heart-rate variability parameter, where the second static heart-rate variability parameter includes a daytime-rest heart-rate variability parameter.

According to an embodiment of the present disclosure, the determining, based on the first mean heart-rate parameters respectively corresponding to the plurality of first time periods, the first static heart-rate parameter includes: determining a minimum first mean heart-rate parameter in the first mean heart-rate parameters respectively corresponding to the plurality of first time periods; and determining the minimum first mean heart-rate parameter as the first static heart-rate parameter.

According to an embodiment of the present disclosure, the determining, based on the first mean heart-rate parameters respectively corresponding to the plurality of first time periods, the first static heart-rate parameter includes: selecting a preset time interval in the first time interval, where a length of the preset time interval is less than a length of the first time interval; determining a minimum first mean heart-rate parameter in the first mean heart-rate parameters respectively corresponding to the first time periods; determining the minimum first mean heart-rate parameter as the first static heart-rate parameter.

According to an embodiment of the present disclosure, before the determining, based on the plurality of first time periods, first mean heart-rate parameters respectively corresponding to the plurality of first time periods, the method further includes: removing an unstable time interval corresponding to each of the plurality of first time periods; where the determining, based on the plurality of first time periods, first mean heart-rate parameters respectively corresponding to the plurality of first time periods includes: determining, based on a plurality of first time periods without the unstable time interval, the first mean heart-rate parameters respectively corresponding to the plurality of first time periods.

According to an embodiment of the present disclosure, the determining, based on the second mean heart-rate parameters respectively corresponding to the plurality of second time periods, the second static heart-rate parameter includes: determining a minimum second mean heart-rate parameter in the second mean heart-rate parameters respectively corresponding to the plurality of second time periods; and determining the minimum second mean heart-rate parameter as the second static heart-rate parameter.

According to an embodiment of the present disclosure, before the determining, based on the plurality of second time periods, second mean heart-rate parameters respectively corresponding to the plurality of second time periods, the method further includes: removing an unstable time interval corresponding to each of the plurality of second time periods; where the determining, based on the plurality of second time periods, second mean heart-rate parameters respectively corresponding to the plurality of second time periods includes: determining, based on a plurality of second time periods without the unstable time interval, the second mean heart-rate parameters respectively corresponding to the plurality of second time periods.

According to another aspect, an embodiment of the present disclosure provides a parameter determination apparatus, including: a first determination module, configured to determine, based on a first time interval, a first parameter set corresponding to a subject to be tested, where the first time interval includes a nighttime interval, the first parameter set includes a first heart-rate characterization parameter and a first heart-rate variability characterization parameter, the first heart-rate characterization parameter includes a first mean heart-rate parameter and a first static heart-rate parameter, and the first heart-rate variability characterization parameter includes a first static heart-rate variability parameter; and a second determination module, configured to determine, based on a second time interval, a second parameter set corresponding to the subject to be tested, where the second time interval includes a daytime interval, the second parameter set includes a second heart-rate characterization parameter and a second heart-rate variability characterization parameter, the second heart-rate characterization parameter includes a second mean heart-rate parameter and a second static heart-rate parameter, and the second heart-rate variability characterization parameter includes a second static heart-rate variability parameter; characterized in that the first time interval includes a plurality of first time periods, the first determination module includes: a first mean heart-rate parameter determination unit, configured to determine, based on the plurality of first time periods, first mean heart-rate parameters respectively corresponding to the plurality of first time periods; a first static heart-rate parameter determination unit, configured to determine, based on the first mean heart-rate parameters respectively corresponding to the plurality of first time periods, the first static heart-rate parameter, where the first static heart-rate parameter includes a sleep rest heart rate parameter; a first static time period determination unit, configured to determine a first time period corresponding to the first static heart-rate parameter as a first static time period; and a first static heart-rate variability parameter determination unit, configured to determine a heart-rate variability parameter corresponding to the first static time period as the first static heart-rate variability parameter, where the first static heart-rate variability parameter includes a sleep rest heart rate variability parameter; the second time interval includes a plurality of second time periods, and second determination module includes: a second mean heart-rate parameter determination unit, configured to determine, based on the plurality of second time periods, second mean heart-rate parameters respectively corresponding to the plurality of second time periods; a second static heart-rate parameter determination unit, configured to determine, based on the second mean heart-rate parameters respectively corresponding to the plurality of second time periods, the second static heart-rate parameter, where the second static heart-rate parameter includes a daytime-rest heart-rate parameter; a third static time period determination unit, configured to determine a second time period corresponding to the second static heart-rate parameter as a third static time period; and a second static heart-rate variability parameter determination unit, configured to determine a heart-rate variability parameter corresponding to the third static time period as the second static heart-rate variability parameter, where the second static heart-rate variability parameter includes a daytime-rest heart-rate variability parameter.

According to another aspect, an embodiment of the present disclosure provides a non-transitory computer-readable storage medium, where a computer program is stored for performing the parameter determination method provided by embodiments described above.

According to another aspect, an embodiment of the present disclosure provides an electronic device, including: a processor; and a memory, configured to store executable instructions of the processor, where the processor is configured to implement the parameter determination method provided by embodiments described above.

According to another aspect, an embodiment of the present disclosure provides a parameter determination method, including: determining, based on a first time interval, a first parameter set corresponding to a subject to be tested, where the first time interval includes a nighttime interval, the first parameter set includes a first heart-rate characterization parameter and a first heart-rate variability characterization parameter, the first heart-rate characterization parameter includes a first mean heart-rate parameter and a first static heart-rate parameter, and the first heart-rate variability characterization parameter includes a first static heart-rate variability parameter; and determining, based on a second time interval, a second parameter set corresponding to the subject to be tested, where the second time interval includes a daytime interval, the second parameter set includes a second heart-rate characterization parameter and a second heart-rate variability characterization parameter, the second heart-rate characterization parameter includes a second mean heart-rate parameter and a second static heart-rate parameter, and the second heart-rate variability characterization parameter includes a second static heart-rate variability parameter; characterized in that the first time interval includes a plurality of first time periods, the determining, based on a first time interval, a first parameter set corresponding to a subject to be tested includes: determining, based on the plurality of first time periods, first heart-rate variability parameters respectively corresponding to the plurality of first time periods; determining, based on the first heart-rate variability parameters respectively corresponding to the plurality of first time periods, the first static heart-rate variability parameter, where the first static heart-rate variability parameter includes a sleep rest heart rate variability parameter; determining a first time period corresponding to the first static heart-rate variability parameter as a first static time period; and determining a first mean heart-rate parameter corresponding to the first static time period as the first static mean heart-rate parameter, where the first static mean heart-rate parameter includes a sleep rest heart rate parameter; the second time interval includes a plurality of second time periods, and the determining, based on a second time interval, a second parameter set corresponding to the subject to be tested includes: determining, based on the plurality of second time periods, second heart-rate variability parameters respectively corresponding to the plurality of second time periods; determining, based on the second heart-rate variability parameters respectively corresponding to the plurality of second time periods, the second static heart-rate variability parameter, where the second static heart-rate variability parameter includes a daytime-rest heart-rate variability parameter; determining a second time period corresponding to the second static heart-rate variability parameter as a third static time period; and determining a second mean heart-rate parameter corresponding to the third static time period as the second static heart-rate parameter, where the second static heart-rate parameter includes a daytime-rest heart-rate parameter.

The parameter determination method provided by the present disclosure reduces an influence of interference factors in a parameter measurement process by determining parameter sets (that is, the first parameter set and the second parameter set) corresponding to the subject to be tested in the first and second time intervals, so that accuracy of an obtained parameter set and an over-time comparability are improved. Moreover, a characterization accuracy and over-time comparability may be effectively improved by using the first and second parameter sets determined by the present disclosure to characterize a level and/or a state of neurohormone.

### BRIEF DESCRIPTION OF THE DRAWINGS

Through a more detailed description of embodiments of the present disclosure with reference to accompanying drawings, the present disclosure described above and other objects, features, and advantages of the present disclosure may become more apparent. The accompanying drawings are used to provide a further understanding of the embodiments of the present disclosure, and constitute a part of specification for explaining the present disclosure together with the embodiments of the present disclosure, and do not constitute a limitation on the present disclosure. In the accompanying drawings, the same reference numerals generally represent the same component or step.
FIG. 1 is a schematic diagram of an applicable scenario of an embodiment of the present disclosure.
FIG. 2 is a schematic diagram of another applicable scenario of an embodiment of the present disclosure.
FIG. 3 is a schematic flowchart of a parameter determination method according to an exemplary embodiment of the present disclosure.
FIG. 4 is a schematic flowchart of a method for determining, based on a first time interval, a first parameter set corresponding to a subject to be tested according to an exemplary embodiment of the present disclosure.
FIG. 5 is a schematic flowchart of a method for determining, based on the first mean heart-rate parameters respectively corresponding to the plurality of first time periods, a first static heart-rate parameter according to an exemplary embodiment of the present disclosure.
FIG. 6 is a schematic flowchart of a method for determining, based on a first time interval, a first parameter set corresponding to a subject to be tested according to another exemplary embodiment of the present disclosure.
FIG. 7 is a schematic flowchart of a method for determining, based on a first time interval, a first parameter set corresponding to a subject to be tested according to still another exemplary embodiment of the present disclosure.
FIG. 8 is a schematic flowchart of a method for determining, based on a second time interval, a second parameter set corresponding to a subject to be tested according to an exemplary embodiment of the present disclosure.
FIG. 9 is a schematic flowchart of a method for determining, based on the second mean heart-rate parameters respectively corresponding to the plurality of second time periods, a second static heart-rate parameter according to an exemplary embodiment of the present disclosure.
FIG. 10 is a schematic flowchart of a method for determining, based on a second time interval, a second parameter set corresponding to a subject to be tested according to another exemplary embodiment of the present disclosure.
FIG. 11 is a schematic structural diagram of a parameter determination apparatus according to an exemplary embodiment of the present disclosure.
FIG. 12 is a schematic structural diagram of a first determination module according to an exemplary embodiment of the present disclosure.
FIG. 13 is a schematic structural diagram of a first determination module according to another exemplary embodiment of the present disclosure.
FIG. 14 is a schematic structural diagram of a first determination module according to still another exemplary embodiment of the present disclosure.
FIG. 15 is a schematic structural diagram of a second determination module according to an exemplary embodiment of the present disclosure.
FIG. 16 is a schematic structural diagram of a second determination module according to another exemplary embodiment of the present disclosure.
FIG. 17 is a schematic structural diagram of an electronic device according to an exemplary embodiment of the present disclosure.

### DETAILED DESCRIPTION OF THE EMBODIMENTS

Hereinafter, exemplary embodiments provided by the present disclosure are described in detail with reference to accompanying drawings. Apparently, described embodiments are only a part, but not all, of the embodiments of the present disclosure, and it should be understood that the present disclosure is not limited by the exemplary embodiments described herein.

### Overview

As is well known, neurohormones driven by sympathetic and parasympathetic nerves are highly susceptible to various factors. Especially for heart disease patients with heart failure or arrhythmia, their neurohormones are highly susceptible to factors such as circadian rhythm, course of disease, sleep status, exercise or activity levels, medication, emotional changes and the like. Therefore, in the prior art, heart rate (HR) and heart rate variability (HRV) are usually used as diagnostic criteria or alternative measurement indicators to characterize neurohormones. Specifically, the heart rate refers to a number of heartbeats per minute. The heart rate variability refers to changes in differences between successive heartbeat cycles. Sources for heart rate calculation include but are not limited to body surface electrocardiogram, endocardium, epicardium electrocardiogram, electrocardiogram from a subcutaneous electrode, or ventricular heart rate or atrial heart rate calculated by R wave and P wave. Moreover, it should be understood that heart rate may also be calculated by using other techniques, such as but not limited to photoplethysmography (PPG).

However, both the heart rate and the heart rate variability are still highly susceptible. For example, the heart rate and/or the heart rate variability often change during different sleep stages at night, and this change may not necessarily be related to neurohormones. Moreover, due to a low heart rate, a patient equipped with a pacemaker or an implantable cardioverter defibrillator (ICD) usually has pace-making at night; in this case, the patient's real heart rate is usually "hidden", so that a real heart rate would not be reflected in the measured heart rate data. Furthermore, in the prior arts, daytime heart rate and daytime heart rate variability measured also include data obtained during exercise and emotional arousal phases. However, the data obtained during exercise and emotional arousal phases may only characterize short-term states, and these short-term changes are different at different times (days). Meanwhile, diseases such as heart failure have a significant impact on the patient's level and/or state of neurohormone, but the impact is relatively long-term in terms of time. It can be seen that there is an urgent need for a parameter determination method to determine parameters that can accurately characterize the level and/or state of neurohormone, and make the parameters determined more comparable.

Based on the aforementioned technical problems, a basic concept of the present disclosure is to propose a parameter determination method, a parameter determination apparatus, a computer-readable storage medium, and an electronic device.

A parameter determination method provided by the present disclosure, includes: determining, based on a first time interval, a first parameter set corresponding to a subject to be tested, where the first time interval includes a nighttime interval, the first parameter set includes a first heart-rate characterization parameter and a first heart-rate variability characterization parameter; and determining, based on a second time interval, a second parameter set corresponding to the subject to be tested, where the second time interval includes a daytime interval, the second parameter set includes a second heart-rate characterization parameter and a second heart-rate variability characterization parameter.

The parameter determination method provided by the present disclosure reduces an influence of interference factors in a parameter measurement process by determining parameter sets (that is, the first parameter set and the second parameter set) corresponding to the subject to be tested in the first and second time intervals, so that accuracy of an obtained parameter set and an over-time comparability are improved. Moreover, a characterization accuracy and over-time comparability may be effectively improved by using the first and second parameter sets determined by the present disclosure to characterize a level and/or a state of neurohormone.

After the basic principles of the present disclosure are introduced, a specific introduction of various non limiting embodiments of the present disclosure is given below with reference to accompanying drawings.

### Exemplary Scenario

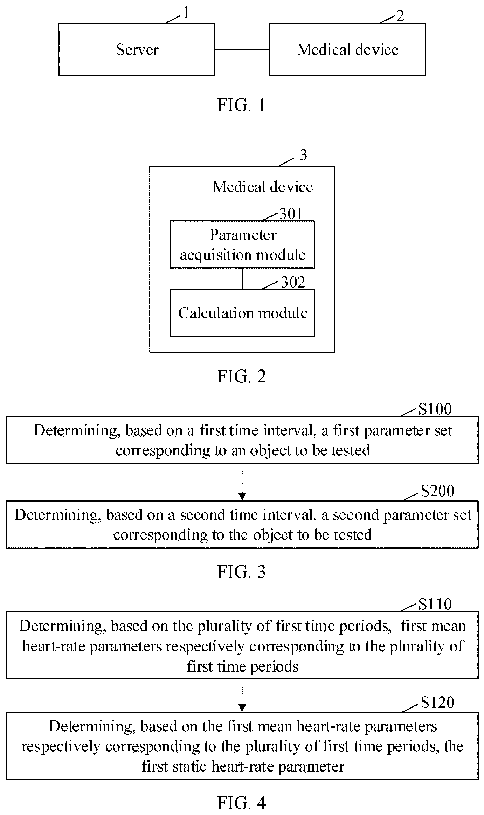

FIG. 1 is a schematic diagram of an applicable scenario of an embodiment of the present disclosure. As shown in FIG. 1, the scenario where embodiments of the present disclosure are applicable to includes a server 1 and a medical device 2. The server 1 and the medical device 2 are connected through communication.

Specifically, the medical device 2 is used to collect a criterion parameter of the subject to be tested, including but not limited to a heart-rate parameter and a heart-rate variability parameter. The server 1 is used to determine a first parameter set corresponding to a subject to be tested based on a first time interval, and to determine a second parameter set corresponding to the subject to be tested based on a second time interval. The first time interval includes a nighttime interval, and the first parameter set includes a first heart-rate characterization parameter and a first heart-rate variability characterization parameter. The second time interval includes a daytime interval, and the second parameter set includes a second heart-rate characterization parameter and a second heart-rate variability characterization parameter. That is, a parameter determination method is implemented in this scenario.

Exemplarily, the server 1 determines the first and/or second parameter sets mentioned above according to the criterion parameters collected by the medical device 2.

As the parameter determination method is realized through the server 1 according to the above scenario shown in FIG. 1, not only adaptability to different scenarios is improved, but also a computational complexity of the medical device 2 is effectively reduced.

It should be noted that the present disclosure also applies to another scenario. FIG. 2 is a schematic diagram of another applicable scenario of an embodiment of the present disclosure. Specifically, this scenario includes a medical device 3, and the medical device 3 includes a parameter acquisition module 301 and a calculation module 302.

Specifically, the parameter acquisition module 301 in the medical device 3 is used to collect criterion parameters of a subject to be tested, including but not limited to a heart-rate parameter and a heart-rate variability parameter. The calculation module 302 is used to determine a first parameter set corresponding to a subject to be tested based on a first time interval, and to determine a second parameter set corresponding to the subject to be tested based on a second time interval. The first time interval includes a nighttime interval, and the first parameter set includes a first heart-rate characterization parameter and a first heart-rate variability characterization parameter. The second time interval includes a daytime interval, and the second parameter set includes a second heart-rate characterization parameter and a second heart-rate variability characterization parameter. That is, a parameter determination method is implemented in this scenario.

Exemplarily, the calculation module 302 determines the first and/or second parameter sets mentioned above according to the criterion parameters collected by the parameter acquisition module 301.

As the parameter determination method is realized through the medical device 3 in the scenario described above shown in FIG. 2, there is no need for data transmission operations with servers and other related devices. Therefore, the above scenario can ensure a real-time performance of the parameter determination method.

It should be noted that the medical devices mentioned in the above scenarios may be either Implantable Medical Devices (IMD) or Wearable Medical Devices (WMD), and are not uniformly limited in the present disclosure.

### Exemplary Method

FIG. 3 is a schematic flowchart of a parameter determination method according to an exemplary embodiment of the present disclosure. As shown in FIG. 3, a parameter determination method provided by an embodiment of the present disclosure includes the following steps.

Step S100: determining, based on a first time interval, a first parameter set corresponding to a subject to be tested.

Exemplarily, the first time interval includes a nighttime interval. For example, in a 24-hour system, the first time interval is from 18:00 of a current day to 6:00 of a next day. Preferably, the first time interval is from 22:00 of a current day to 6:00 of a next day. Preferably, during the first time interval, the subject to be tested is in a resting or sleeping state to reduce interference, thereby improving accuracy of the first parameter set obtained finally. The resting state refers to a state in which a body is at rest, while the sleeping state refers to a state of being asleep, where a body is still, and in a "lying" posture.

Exemplarily, the subject to be tested mentioned in Step S100 refers to a human body. That is, a first parameter set corresponding to a human body is determined. It should be understood that the determining a first parameter set corresponding to a subject to be tested mentioned in Step S100 may mean calculating the first parameter set corresponding to the subject to be tested.

According to an embodiment of the present disclosure, the first parameter set includes a first heart-rate characterization parameter and a first heart-rate variability characterization parameter. The first heart-rate characterization parameter refers to a parameter that can characterize a heart-rate situation in the first time interval. The first heart-rate variability characterization parameter refers to a parameter that can characterize a heart-rate variability in the first time interval. For example, the first heart-rate characterization parameter is obtained based on a heart rate measured in real time, and the first heart-rate variability characterization parameter is obtained based on a heart-rate variability measured in real time.

Step S200: determining, based on a second time interval, a second parameter set corresponding to the subject to be tested.

Exemplarily, the second time interval includes a daytime interval. For example, in the 24-hour system, the second time interval is from 6:00 to 18:00 of a current day. Preferably, the second time interval is from 8:00 to 20:00 of a current day. Preferably, during the second time interval, the subject to be tested is in a resting state, or almost has no movement or activity to reduce interference, thereby improving accuracy and comparability of the second parameter set obtained finally. More preferably, during the second time interval, the subject to be tested is at rest or has no movement in a non-standing position. Exemplarily, a non-motion state mentioned above may be determined through some relevant motion/body movement or other physiological parameter thresholds. For example, if a real-time motion signal of the subject to be tested is below a certain threshold, or if a real-time heart rate of the subject to be tested does not exceed a preset exercise heart-rate threshold, it can be determined that the subject to be tested is at the non-motion state.

According to an embodiment of the present disclosure, the second parameter set includes a second heart-rate characterization parameter and a second heart-rate variability characterization parameter. The second heart-rate characterization parameter refers to a parameter that can characterize a heart-rate situation in the second time interval. The second heart-rate variability characterization parameter refers to a parameter that can characterize a heart-rate variability in the second time interval. For example, the second heart-rate characterization parameter is obtained based on a heart rate measured in real time, and the second heart-rate variability characterization parameter is obtained based on a heart-rate variability measured in real time.

In practical applications, based on the first time interval, the first parameter set corresponding to a subject to be tested is determined; and based on the second time interval, the second parameter set corresponding to the subject to be tested is determined.

The parameter determination method provided by the present disclosure reduces an influence of interference factors in a parameter measurement process by determining parameter sets (that is, the first parameter set and the second parameter set) corresponding to the subject to be tested in the first and second time intervals, so that accuracy of an obtained parameter set and an over-time comparability are improved. Moreover, a characterization accuracy may be effectively improved by using the first and second parameter sets determined by the present disclosure to characterize a level and/or a state of neurohormone.

FIG. 4 is a schematic flowchart of a method for determining, based on a first time interval, a first parameter set corresponding to a subject to be tested according to an exemplary embodiment of the present disclosure. The embodiment shown in FIG. 4 is an extension of the embodiment shown in FIG. 3. Differences between the embodiment shown in FIG. 4 and the embodiment shown in FIG. 3 will be introduced below, and similarities will not be described herein again.

As shown in FIG. 4, according to a parameter determination method provided by the present disclosure, the first time interval includes a plurality of first time periods, and the first heart-rate characterization parameter includes a first mean heart-rate parameter and a first static heart-rate parameter. And the determining, based on a first time interval, a first parameter set corresponding to a subject to be tested includes the following steps.

Step S110: determining, based on the plurality of first time periods, first mean heart-rate parameters respectively corresponding to the plurality of first time periods.

Exemplarily, the first time period is a 60 second time window. In other words, Step S110 refers to determining, based on a plurality of time windows included in the first time interval, the first mean heart-rate parameters respectively corresponding to the plurality of time windows.

Optionally, the first mean heart-rate (MHR) parameter corresponding to each of the plurality of time windows refers to a mean heart rate measured during a corresponding time window.

Step S120: determining, based on the first mean heart-rate parameters respectively corresponding to the plurality of first time periods, the first static heart-rate parameter.

According to an embodiment of the present disclosure, the first static heart-rate parameter refers to a sleep rest heart rate (Sleep RHR) parameter.

As the first static heart-rate parameter is determined based on the first mean heart-rate parameters respectively corresponding to the plurality of first time periods, the first static heart-rate parameter obtained may fully consider characteristics of the first mean heart-rate parameters respectively corresponding to the plurality of the first time periods. Thus, the first static heart-rate parameter obtained may better characterize the heart rate situation of the subject to be tested in the first time interval.

According to the parameter determination method provided by the embodiment of the present disclosure, by determining, based on the plurality of first time periods, the first mean heart-rate parameters respectively corresponding to the plurality of first time periods and determining, based on the first mean heart-rate parameters respectively corresponding to the plurality of first time periods, the first static heart-rate parameter, the first parameter set corresponding to the subject to be tested is determined based on the first time interval. Compared with real-time heart rate, the first mean heart-rate parameter and the first static heart-rate parameter may more accurately characterize the heart rate situation of the subject to be tested in the first time interval. Therefore, when the first mean heart-rate parameter and the first static heart-rate parameter obtained according to the embodiment of the present disclosure are used to predict the neurohormone level and/or state of the subject to be tested in the first time interval, a more accurate prediction result of the neurohormone may be obtained.

According to another embodiment of the present disclosure, a process of determining the first mean heart-rate parameter includes: determining, based on the plurality of first time periods, heart-rate variability parameters respectively corresponding to the plurality of first time periods; and determining, based on the heart-rate variability parameters respectively corresponding to the plurality of first time periods, the first mean heart-rate parameter. For example, firstly, a first time period corresponding to a maximum heart-rate variability parameter is determined from the heart-rate variability parameters corresponding to the plurality of first time periods, and then, a mean heart-rate parameter corresponding to the first time period which corresponds to the maximum heart-rate variability parameter is determined to be the first mean heart-rate parameter. Other statistical descriptions may also be used here for the heart-rate variability parameters, such as a 75% value. In addition, it should be noted that the mean heart-rate parameters may also be described in other statistical methods, such as a 25% value.

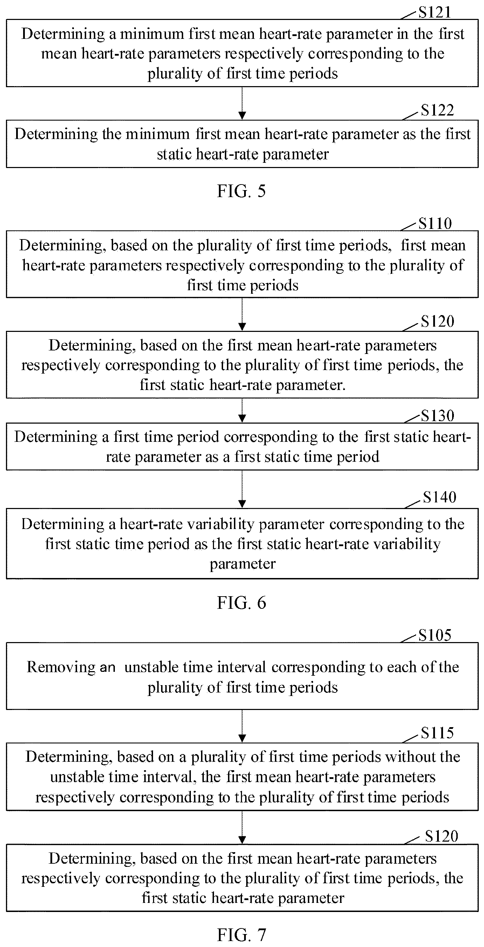

Similarly, according to an embodiment of the present disclosure, an unstable time interval may be removed from the first time interval first. A specific meaning of the unstable time interval may be referred to an embodiment shown in FIG. 7, and will not be described herein again.

FIG. 5 is a schematic flowchart of a method for determining, based on the first mean heart-rate parameters respectively corresponding to the plurality of first time periods, the first static heart-rate parameter according to an exemplary embodiment of the present disclosure. The embodiment shown in FIG. 5 is an extension of the embodiment shown in FIG. 4. Differences between the embodiment shown in FIG. 5 and the embodiment shown in FIG. 4 will be introduced below, and similarities will not be described herein again.

As shown in FIG. 5, according to a parameter determination method provided by the present disclosure, the determining, based on the first mean heart-rate parameters respectively corresponding to the plurality of first time periods, the first static heart-rate parameter includes the following steps.

Step S121: determining a minimum first mean heart-rate parameter in the first mean heart-rate parameters respectively corresponding to the plurality of first time periods.

Step S122: determining the minimum first mean heart-rate parameter as the first static heart-rate parameter.

For example, the first time interval includes five first time periods, which are first time period a, first time period b, first time period c, first time period d, and first time period e respectively. Correspondingly, a first mean heart-rate parameter corresponding to the first time period a is 80 beats/minute, a first mean heart-rate parameter corresponding to the first time period b is 85 beats/minute, a first mean heart-rate parameter corresponding to the first time period c is 90 beats/minute, a first mean heart-rate parameter corresponding to the first time period d is 75 beats/minute, and a first mean heart-rate parameter corresponding to the first time period e is 95 beats/minute. Then, the minimum first mean heart-rate parameter is the first mean heart-rate parameter corresponding to the first time period d (75 beats/minute). Therefore, as described in Step S122, the first mean heart-rate parameter corresponding to the first time period d (75 beats/minute) may be determined as the first static heart-rate parameter.

According to the parameter determination method provided by the embodiment of the present disclosure, by determining the minimum first mean heart-rate parameter in the first mean heart-rate parameters respectively corresponding to the plurality of first time periods; and determining the minimum first mean heart-rate parameter as the first static heart-rate parameter, the first static heart-rate parameter may be determined based on the first mean heart-rate parameters respectively corresponding to the plurality of first time periods. As the minimum first mean heart-rate parameter in the first mean heart-rate parameters respectively corresponding to the plurality of first time periods may characterize, to some extent, a heart-rate characteristic of the subject to be tested during the first time interval, the embodiment of the present disclosure may further enrich effective information contained in the first parameter set, thereby improving subsequent prediction accuracy and comparability of neurohormone level and/or state.

Another embodiment of the present disclosure is extended from the embodiment shown in FIG. 5. According to the embodiment, before the determining a minimum first mean heart-rate parameter between the first mean heart-rate parameters respectively corresponding to the plurality of first time periods, the method further includes: selecting a preset time interval in the first time interval, where a length of the preset time interval is less than a length of the first time interval. And according to the embodiment, the determining a minimum first mean heart-rate parameter in the first mean heart-rate parameters respectively corresponding to the plurality of first time periods includes: determining a minimum first mean heart-rate parameter in the first mean heart-rate parameters respectively corresponding to the first time periods included in the preset time interval. In this way, mean heart-rate parameters corresponding to some atypical time intervals in the first time interval may be effectively removed, so that accuracy of the minimum first mean heart-rate parameter may be further improved.

FIG. 6 is a schematic flowchart of a method for determining, based on a first time interval, a first parameter set corresponding to a subject to be tested according to another exemplary embodiment of the present disclosure. The embodiment shown in FIG. 6 is an extension of the embodiment shown in FIG. 4. Differences between the embodiment shown in FIG. 6 and the embodiment shown in FIG. 4 will be introduced below, and the similarities will not be described herein again.

As shown in FIG. 6, according to a parameter determination method provided by the present disclosure, after the determining, based on the first mean heart-rate parameters respectively corresponding to the plurality of first time periods, the first static heart-rate parameter, the method further includes the following steps.

Step S130: determining a first time period corresponding to the first static heart-rate parameter as a first static time period.

Step S140: determining a heart-rate variability parameter corresponding to the first static time period as the first static heart-rate variability parameter.

According to an embodiment of the present disclosure, the first static heart-rate variability parameter refers to a sleep rest heart rate variability (Sleep RHRV) parameter.

In practical applications, the first mean heart-rate parameters respectively corresponding to the plurality of first time periods are determined based on the plurality of first time periods; the first static heart-rate parameter is then determined based on the first mean heart-rate parameters respectively corresponding to the plurality of first time periods; and the first time period corresponding to the first static heart-rate parameter is determined as the first static time period. A heart-rate variability parameter corresponding to the first static time period is determined as the first static heart-rate variability parameter; and then a second parameter set corresponding to the subject to be tested is determined based on a second time interval.

As the first static heart-rate variability parameter is determined based on the first static heart-rate parameter, an ability and comparability of characterizing a neurohormone level and/or state of a subject to be tested during the first time interval may be improved compared with the prior arts.

It should be noted that in another embodiment of the present disclosure, the first static heart-rate variability parameter may also be determined first, and then the first static heart-rate parameter is determined (this also applies to the second static heart-rate variability parameter and the second static heart-rate parameter, in other words, the second static heart-rate variability parameter may be determined first, and then the second static heart-rate parameter is determined). For example, based on the plurality of first time periods, first heart-rate variability parameters respectively corresponding to the first time periods are determined; based on the first heart-rate variability parameters respectively corresponding to the plurality of first time periods, a first static heart-rate variability parameter is determined; a first time period corresponding to the first static heart-rate variability parameter is determined as a first static time period; and a mean heart-rate parameter corresponding to the first static time period is determined as the first static heart-rate parameter. The step of determining, based on the first heart-rate variability parameters respectively corresponding to the plurality of first time periods, the first static heart-rate variability parameter may be executed as determining, based on a maximum first heart-rate variability parameter in the first heart-rate variability parameters respectively corresponding to the plurality of first time periods, the first static heart-rate variability parameter. Other statistical descriptions besides a maximum value may also be used here for heart-rate variability parameters, such as a 75% value. In addition, it should be noted that the mean heart-rate parameters may also be described in other statistical methods, such as a 25% value.

FIG. 7 is a schematic flowchart of a method for determining, based on a first time interval, a first parameter set corresponding to a subject to be tested according to still another exemplary embodiment of the present disclosure. The embodiment shown in FIG. 7 is an extension of the embodiment shown in FIG. 4. Differences between the embodiment shown in FIG. 7 and the embodiment shown in FIG. 4 will be introduced below, and the similarities will not be described herein again.

As shown in FIG. 7, according to an parameter determination method provided by the present disclosure, before the determining, based on the plurality of first time periods, first mean heart-rate parameters respectively corresponding to the plurality of first time periods, the method further includes the following steps.

Step S105: removing an unstable time interval corresponding to each of the plurality of first time periods.

Exemplarily, the unstable time interval in Step S105 refers to, for example, a time interval of snoring and/or a time interval of unstable respiratory rate (such as, sleep apnea).

According to an embodiment of the present disclosure, the unstable time interval also includes a time interval corresponding to special stages of sleep with high physical activity. The special stage of sleep is, for example, a Rapid Eye Movement (REM) stage, or a stage when heart rate significant changes during dreaming. And the unstable time interval also includes, for example, time intervals respectively corresponding to events such as premature atrial premature contraction (PAC), premature ventricular contraction (PVC) and atrial tachycardia or atrial arrhythmia (such as atrial fibrillation), and ventricular tachycardia (VT).

And according to the parameter determination method provided by the present disclosure, the determining, based on the plurality of first time periods, first mean heart-rate parameters respectively corresponding to the plurality of first time periods includes the following steps.

Step S115: determining, based on a plurality of first time periods without the unstable time interval, the first mean heart-rate parameters respectively corresponding to the plurality of first time periods.

As specificity of the unstable time is relatively high, which cannot effectively characterize typical situations of the subject to be tested, the embodiment of the present disclosure may improve a characterization ability of the first mean heart-rate parameter and first static heart-rate variability parameter determined by removing the unstable time interval.

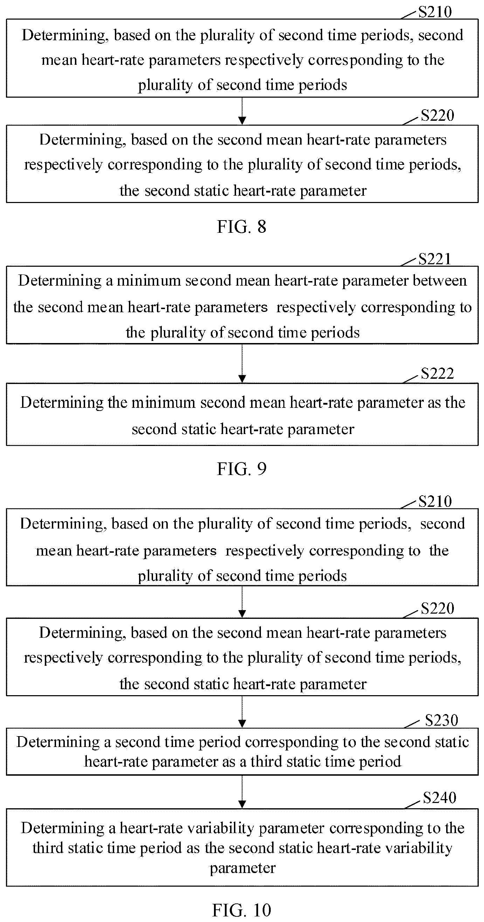

FIG. 8 is a schematic flowchart of a method for determining, based on a second time interval, a second parameter set corresponding to a subject to be tested according to an exemplary embodiment of the present disclosure. The embodiment shown in FIG. 8 is an extension of the embodiment shown in FIG. 3. Differences between the embodiment shown in FIG. 8 and the embodiment shown in FIG. 3 will be introduced below, and the similarities will not be described herein again.

As shown in FIG. 8, according to a parameter determination method provided by the present disclosure, the second time interval includes a plurality of second time periods. And the determining, based on a second time interval, a second parameter set corresponding to the subject to be tested includes the following steps.

Step S210: determining, based on the plurality of second time periods, second mean heart-rate parameters respectively corresponding to the plurality of second time periods.

Exemplarily, the second time period is a 60 second time window. In other words, Step S210 refers to determining, based on a plurality of time windows included in the second time interval, the second mean heart-rate parameters respectively corresponding to the plurality of time windows.

Optionally, the second mean heart-rate (MHR) parameter corresponding to each of the plurality of time windows refers to a mean heart rate measured during a corresponding time window.

Step S220: determining, based on the second mean heart-rate parameters respectively corresponding to the plurality of second time periods, the second static heart-rate parameter.

According to an embodiment of the present disclosure, the second static heart-rate parameter refers to a day rest heart rate (Day RHR) parameter.

As the second static heart-rate parameter is determined based on the second mean heart-rate parameters respectively corresponding to the plurality of second time periods, the second static heart-rate parameter obtained may fully consider characteristics of the second mean heart-rate parameters respectively corresponding to the plurality of the second time periods. Thus, the second static heart-rate parameter obtained may better characterize the heart rate situation of the subject to be tested in the second time interval.

According to the parameter determination method provided by an embodiment of the present disclosure, by determining, based on the plurality of second time periods, second mean heart-rate parameters respectively corresponding to the plurality of second time periods and determining, based on the second mean heart-rate parameters respectively corresponding to the plurality of second time periods, the second static heart-rate parameter, the second parameter set corresponding to the subject to be tested s determined based on the second time interval. Compared with real-time heart rate, the second mean heart-rate parameter and the second static heart-rate parameter may more accurately characterize the heart rate situation of the subject to be tested in the second time interval. Therefore, when the second mean heart-rate parameter and the second static heart-rate parameter obtained according to the embodiment of the present disclosure are used to predict the neurohormone level and/or state of the subject to be tested in the second time interval, a more accurate prediction result of the neurohormone may be obtained.

FIG. 9 is a schematic flowchart of a method for determining, based on the second mean heart-rate parameters respectively corresponding to the plurality of second time periods, the second static heart-rate parameter according to an exemplary embodiment of the present disclosure. The embodiment shown in FIG. 9 is an extension of the embodiment shown in FIG. 8. Differences between the embodiment shown in FIG. 9 and the embodiment shown in FIG. 8 will be introduced below, and the similarities will not be described herein again.

As shown in FIG. 9, according to a parameter determination method provided by the present disclosure, the determining, based on the second mean heart-rate parameters respectively corresponding to the plurality of second time periods, the second static heart-rate parameter includes the following steps.

Step S221: determining a minimum second mean heart-rate parameter between the second mean heart-rate parameters respectively corresponding to the plurality of second time periods.

Step S222: determining the minimum second mean heart-rate parameter as the second static heart-rate parameter.

For example, the second time interval includes 3 second time periods, which are second time period a, second time period b, second time period c respectively. Correspondingly, a second mean heart-rate parameter corresponding to the second time period a is 80 beats/minute, a second mean heart-rate parameter corresponding to the second time period b is 85 beats/minute, and a second mean heart-rate parameter corresponding to the second time period c is 90 beats/minute. Then, the minimum second mean heart-rate parameter is the second mean heart-rate parameter corresponding to the second time period a (80 beats/minute). Therefore, as described in Step S222, the second mean heart-rate parameter corresponding to the second time period a (80 beats/minute) may be determined as the second static heart-rate parameter. It should be noted that other statistical descriptions may also used for the second static heart-rate parameter, such as a 25% value.

For the same reason, as the minimum second mean heart-rate parameter in the second mean heart-rate parameters respectively corresponding to the plurality of second time periods may characterize, to some extent, a heart-rate characteristic of the subject to be tested during the second time interval, the embodiment of the present disclosure may further enrich effective information contained in the second parameter set, thereby improving subsequent prediction accuracy and comparability of neurohormone level and/or state.

Another embodiment of the present disclosure is extended from the embodiment shown in FIG. 8. According to the embodiment, before the determining, based on the plurality of second time periods, second mean heart-rate parameters respectively corresponding to the plurality of second time periods, the method further includes: removing an unstable time interval corresponding to each of the plurality of second time periods. And the determining, based on the plurality of second time periods, second mean heart-rate parameters respectively corresponding to the plurality of second time periods includes: determining, based on a plurality of second time periods without the unstable time interval, the second mean heart-rate parameters respectively corresponding to the plurality of second time periods.

For example, the subject to be tested may be emotionally excited (due to arguments or other reasons) and so on. And the unstable time interval also includes, for example, a time intervals respectively corresponding to events such as premature atrial premature contraction (PAC), premature ventricular contraction (PVC) and atrial tachycardia or atrial arrhythmia (such as atrial fibrillation), and ventricular tachycardia (VT). Therefore, the subsequent prediction accuracy and comparability of neurohormone level and/or state may be further improved. The specific meaning of the unstable time interval may be referred to the embodiment shown in FIG. 7, and will not be described herein again.

FIG. 10 is a schematic flowchart of a method for determining, based on a second time interval, a second parameter set corresponding to a subject to be tested according to another exemplary embodiment of the present disclosure. The embodiment shown in FIG. 10 is an extension of the embodiment shown in FIG. 8. Differences between the embodiment shown in FIG. 10 and the embodiment shown in FIG. 8 will be introduced below, and the similarities will not be described herein again.

As shown in FIG. 10, according to a parameter determination method provided by the present disclosure, the second heart-rate variability characterization parameter includes a second static heart-rate variability parameter; and after the determining, based on the second mean heart-rate parameters respectively corresponding to the plurality of second time periods, the second static heart-rate parameter, the method further includes the following steps.

Step S230: determining a second time period corresponding to the second static heart-rate parameter as a third static time period.

Step S240: determining a heart-rate variability parameter corresponding to the third static time period as the second static heart-rate variability parameter.

According to an embodiment of the present disclosure, the second static heart-rate variability parameter refers to a day rest heart rate variability (Day RHRV) parameter.

In practical applications, the second parameter set corresponding to a subject to be tested is determined based on the second time interval; the second mean heart-rate parameters respectively corresponding to the plurality of second time periods are determined based on the plurality of second time periods; and the second static heart-rate parameter is then determined based on the second mean heart-rate parameters respectively corresponding to the plurality of second time periods. A second time period corresponding to the second static heart-rate parameter is determined as the third static time period; and then a heart-rate variability parameter corresponding to the third static time period is determined as the second static heart-rate variability parameter.

As the second static heart-rate variability parameter is determined based on the second static heart-rate parameter, an ability and comparability of characterizing a neurohormone level and/or state of a subject to be tested during the second time interval, and comparability of the second static heart-rate variability parameter may be improved compared with the prior arts.

According to an embodiment of the present disclosure, the first mean heart-rate parameter and/or the second mean heart-rate parameter mentioned in the above embodiments may be deecribed in other statistical methods, such as median and/or quartile (such as 75% or 25%) of corresponding heart rate and other statistical index parameters. Alternatively, on the basis of the above embodiments, corresponding statistical index parameters such as median and/or quartile (such as 75% or 25%) of heart rate corresponding to the first parameter set and/or the second parameter set may be added. In this way, flexibility of the parameter determination method may be improved and effective information contained in the corresponding parameter set may be enriched.

According to an embodiment of the present disclosure, the first heart-rate variability characterization parameter mentioned in the above embodiments includes a corresponding heart-rate variability parameter, and/or, the second heart-rate variability characterization parameter mentioned in the above embodiments includes but is not limited to commonly used heart-rate variability parameters in literatures. Moreover, the included heart-rate variability parameters are obtained through standard deviation information of heart rate (during R-R interval or P-P interval).

Another embodiment of the present disclosure is extended from the embodiment shown in FIG. 3. According to the embodiment, after the determining, based on a second time interval, a second parameter set corresponding to the subject to be tested, the method further includes: determining, based on the first parameter set and the second parameter set, a level and/or a state of neurohormone corresponding to the subject to be tested.

The embodiment of the present disclosure may improve a characterization accuracy of neurohormone level and/or state by means of the first parameter set and the second parameter set, so that a prerequisite may be provided for better assisting doctors in disease diagnosis operations.

### Exemplary apparatus

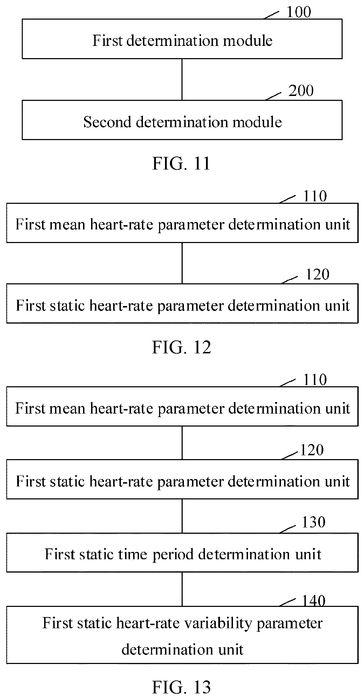

FIG. 11 is a schematic structural diagram of a parameter determination apparatus according to an exemplary embodiment of the present disclosure. As shown in FIG. 11, the parameter determination apparatus provided by the embodiment of the present disclosure includes:
a first determination module 100, configured to determine, based on a first time interval, a first parameter set corresponding to a subject to be tested; and
a second determination module 200, configured to determine, based on a second time interval, a second parameter set corresponding to the subject to be tested.

FIG. 12 is a schematic structural diagram of a first determination module according to an exemplary embodiment of the present disclosure. The embodiment shown in FIG. 12 is an extension of the embodiment shown in FIG. 11. Differences between the embodiment shown in FIG. 12 and the embodiment shown in FIG. 11 will be introduced below, and the similarities will not be described herein again.

As shown in FIG. 12, the first determination module 100 in the parameter determination apparatus provided by the embodiment of the present disclosure includes:
a first mean heart-rate parameter determination unit 110, configured to determine, based on the plurality of first time periods, first mean heart-rate parameters respectively corresponding to the plurality of first time periods; and
a first static heart-rate parameter determination unit 120, configured to determine, based on the first mean heart-rate parameters respectively corresponding to the plurality of first time periods, a first static heart-rate parameter.

According to an embodiment of the present disclosure, the first static heart-rate parameter determination unit 120 is further configured to determine a minimum first mean heart-rate parameter in the first mean heart-rate parameters respectively corresponding to the plurality of first time periods; and determine the minimum first mean heart-rate parameter as the first static heart-rate parameter.

FIG. 13 is a schematic structural diagram of a first determination module according to another exemplary embodiment of the present disclosure. The embodiment shown in FIG. 13 is an extension of the embodiment shown in FIG. 12. Differences between the embodiment shown in FIG. 13 and the embodiment shown in FIG. 12 will be introduced below, and the similarities will not be described herein again.

As shown in FIG. 13, the first determination module 100 in the parameter determination apparatus provided by the embodiment of the present disclosure further includes:
a first static time period determination unit 130, configured to determine a first time period corresponding to the first static heart-rate parameter as a first static time period;
a first static heart-rate variability parameter determination unit 140, configured to determine a heart-rate variability parameter corresponding to the first static time period as the first static heart-rate variability parameter.

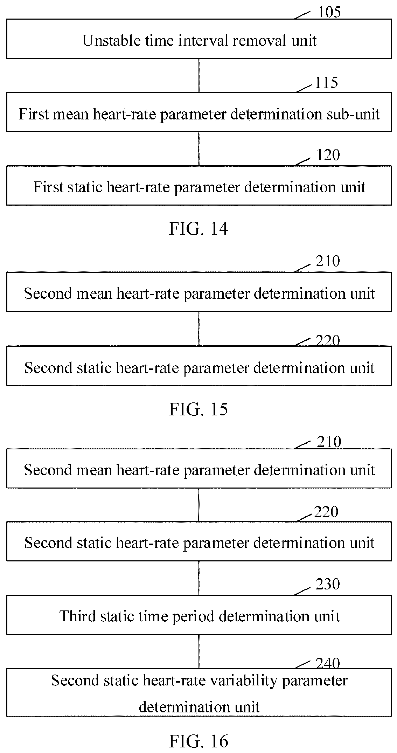

FIG. 14 is a schematic structural diagram of a first determination module according to still another exemplary embodiment of the present disclosure. The embodiment shown in FIG. 14 is an extension of the embodiment shown in FIG. 12. Differences between the embodiment shown in FIG. 14 and the embodiment shown in FIG. 12 will be introduced below, and the similarities will not be described herein again.

As shown in FIG. 14, the parameter determination apparatus provided by an embodiment of the present disclosure further includes:
an unstable time interval removal unit 105, configured to remove an unstable time interval corresponding to each of the plurality of first time periods.

Moreover, the first mean heart-rate parameter determination unit 110 in the parameter determination apparatus provided by an embodiment of the present disclosure includes:
a first mean heart-rate parameter determination sub-unit 115, configured to determine, based on a plurality of first time periods without the unstable time interval, the first mean heart-rate parameters respectively corresponding to the plurality of first time periods.

FIG. 15 is a schematic structural diagram of a second determination module according to an exemplary embodiment of the present disclosure. The embodiment shown in FIG. 15 is an extension of the embodiment shown in FIG. 12. Differences between the embodiment shown in FIG. 15 and the embodiment shown in FIG. 12 will be introduced below, and the similarities will not be described herein again.

As shown in FIG. 15, the second determination module 200 in the parameter determination apparatus provided by an embodiment of the present disclosure includes:
a second mean heart-rate parameter determination unit 210, configured to determine, based on the plurality of second time periods, second mean heart-rate parameters respectively corresponding to the plurality of second time periods;
a second static heart-rate parameter determination unit 220, configured to determine, based on the second mean heart-rate parameters respectively corresponding to the plurality of second time periods, the second static heart-rate parameter.

According to an embodiment of the present disclosure, the second static heart-rate parameter determination unit 220 is further configured to determine a minimum second mean heart-rate parameter between the second mean heart-rate parameters respectively corresponding to the plurality of second time periods; and determine the minimum second mean heart-rate parameter as the second static heart-rate parameter.

FIG. 16 is a schematic structural diagram of a second determination module according to another exemplary embodiment of the present disclosure. The embodiment shown in FIG. 16 is an extension of the embodiment shown in FIG. 15. Differences between the embodiment shown in FIG. 16 and the embodiment shown in FIG. 15 will be introduced below, and the similarities will not be described herein again.

As shown in FIG. 16, the second determination module 200 in the parameter determination apparatus provided by an embodiment of the present disclosure further includes:
a third static time period determination unit 230, configured to determine a second time period corresponding to the second static heart-rate parameter as a third static time period;
a second static heart-rate variability parameter determination unit 240, configured to determine a heart-rate variability parameter corresponding to the third static time period as the second static heart-rate variability parameter.

It should be understood that operations and functions, including the first determination module 100 and the second determination module 200; the first mean heart-rate parameter determination unit 110, the first static heart-rate parameter determination unit 120, the first static time period determination unit 130, and the first static heart-rate variability parameter determination unit 140 included in the first determination module 100; the second mean heart-rate parameter determination unit 210, the second static heart-rate parameter determination unit 220, the third static time period determination unit 230, and the second static heart-rate variability parameter determination unit 240 included in the second determination module 200 included in the parameter determination apparatus provided in FIG. 11 to FIG. 16 may be referred to the parameter determination method provided in FIG. 3 to FIG. 10 above, and the similarities will not be described herein again.

Moreover, it should be noted that the parameter determination apparatus mentioned in the above embodiments may combine the parameter determination method with existing medical equipment/device to realize the parameter determination method mentioned in the above embodiments by using criterion parameters collected by a data collection function of the existing medical equipment/device and/or a judgment function of the existing medical equipment/device; and then, the purpose of defining and detecting arrhythmia events (including but not limited to PVC or PAC, non persistent VT, atrial fibrillation (AF), ventricular tachycardia (VT), and ventricular fibrillation (VF)) is realized through the first parameter set and second parameter set determined by the parameter determination method.

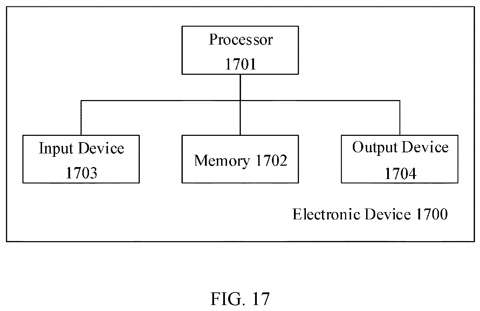

An electronic device according to an embodiment of the present disclosure will be described with reference to FIG. 17. FIG. 17 is a schematic structural diagram of an electronic device according to an exemplary embodiment of the present disclosure.

As shown in FIG. 17, the electronic device 1700 includes: one or more processors 1701 and a memory 1702.

The processor 1701 may be a central processing unit (CPU) or other form of processing unit with data transmission capability and/or instruction execution capability, and may control other components in the electronic device 1700 to perform a desired function.

The memory 1702 may include one or more computer program products, which may include various forms of computer-readable storage media, such as volatile memory and/or non-volatile memory. The volatile memory may include, for example, random access memory (RAM) and/or cache memory (Cache). The non-volatile memory may include, for example, read only memory (ROM), hard disk, flash memory and so on. One or more computer program instructions may be stored in a computer-readable storage medium, and the processor 1701 may run the program instructions to realize the steps and/or other desired functions of the parameter determination method according to the embodiments of the present disclosure. And the computer-readable storage medium may further store various contents such as heart rate information.

In an embodiment, the electronic device 1700 may also include an input device 1703 and an output device 1704. These components are interconnected by a bus system and/or other forms of connection mechanism (not shown).

The input device 1703 may also include, for example, a keyboard, a mouse, and so on.

The output device 1704 may output various kinds of information, including first parameter set and the second parameter set determined, to the outside. The output device 1704 may include, for example, a display, a speaker, a printer, a communication network and remote output device connected on it, and so on.

To simplify, only some of the components related to the present disclosure in the electronic device 1700 are shown in FIG. 17 and components such as bus, input/output interface and so on are omitted. Moreover, the electronic device 1700 may also include any other appropriate components according to the specific disclosure.

Beyond the above methods and devices, the embodiments of the present disclosure may also be computer program products, including computer program instructions, which enable the processor to perform the steps of the parameter determination method as described in any one of above embodiments when run by the processor.

The computer program product may write program code for executing the operation of the embodiments of the present disclosure in any combination of one or more programming languages. The programming languages include object-oriented programming languages, such as Java, C++, and also include conventional procedural programming languages, such as "C" language or similar programming languages. The program code may be completely executed on the user's computing device, partially executed on the user's device, executed as an independent software package, partially executed on the user's computing device and partially executed on the remote computing device, or completely executed on the remote computing device or processor.

Moreover, the embodiments of the present disclosure may also be a computer-readable storage medium on which computer executable instructions are stored. When the computer executable instructions are run by the processor, the processor performs the steps of the parameter determination method described in the "exemplary method" according to the embodiments of the present disclosure.

The computer-readable storage medium may adopt any combination of one or more readable media. The readable medium may be a readable signal medium or a readable storage medium. The readable storage medium may include, for example, but not limited to, systems, apparatuses or means of electricity, magnetism, light, electromagnetism, infrared ray, or semiconductor, or any combination of the above. More specific examples of readable storage media (not an exhaustive list) include: electronic connection with one or more wires, portable disk, hard disk, RAM, ROM, erasable programmable read only memory (EPROM) or flash memory, optical fiber, compact disk read only memory (CD-ROM), optical storage means, magnetic storage means, or any suitable combination of the above.

The above describes the basic principle of the present disclosure with reference to specific embodiments. However, it should be noted that the advantages, superiorities, effects and so on mentioned in the present disclosure are only examples, but not limitations. It cannot be considered that these advantages, superiorities, effects and so on are necessary for each embodiment of the present disclosure. In addition, the specific details disclosed above are only for the purpose of example and easy understanding, but not for limitation. The present disclosure is not limited to the above specific details.

The block diagrams of means, apparatuses, devices and systems involved in the present disclosure are only illustrative examples and are not intended to require or imply that they must be connected, disposed and configured in the manner shown in the block diagram. As those skilled in the art will recognize, these means, apparatuses, devices and systems can be connected, disposed and configured in any way. The terms such as "include", "contain", "have" and so on are open-class words, and referring to "include but not limited to", and can be used interchangeably. The terms "or" and "and" refer to the terms "and/or" and can be used interchangeably, unless the context clearly indicate otherwise. The term "such as" refers to the terms "such as but not limited to" and can be used interchangeably.

It should also be noted that each component or step in the apparatus, device and method of the present disclosure can be **decomposed and/or reassembled.**

The above description of the disclosed aspects is provided to enable any person skilled in the art to make or use the present disclosure. Any modification to these aspects is obvious to a person skilled in the art, and the general principles defined herein can be applied to other aspects without departing from the protection scope of the present disclosure. Therefore, the present disclosure is not intended to be limited to the aspects shown herein, but to the widest range consistent with the principles and novel features disclosed herein.

The above description has been given for the purpose of illustration and description. In addition, the description is not intended to limit the embodiments of the present disclosure to the form disclosed herein. Although several example aspects and embodiments have been discussed above, a person skilled in the art may recognize certain variations, modifications, changes, additions and sub-combinations thereof.

## Claims

1. A parameter determination method, implemented by a server or a medical device, comprising:
determining (S100), based on a first time interval, a first parameter set corresponding to a subject to be tested, wherein the first time interval comprises a nighttime interval, the first parameter set comprises a first heart-rate characterization parameter and a first heart-rate variability characterization parameter, the first heart-rate characterization parameter comprises a first mean heart-rate parameter and a first static heart-rate parameter, and the first heart-rate variability characterization parameter comprises a first static heart-rate variability parameter; and
determining (S200), based on a second time interval, a second parameter set corresponding to the subject to be tested, wherein the second time interval comprises a daytime interval, the second parameter set comprises a second heart-rate characterization parameter and a second heart-rate variability characterization parameter, the second heart-rate characterization parameter comprises a second mean heart-rate parameter and a second static heart-rate parameter, and the second heart-rate variability characterization parameter comprises a second static heart-rate variability parameter;
the first time interval comprises a plurality of first time periods, the determining, based on a first time interval, a first parameter set corresponding to a subject to be tested comprises:
determining (S110), based on the plurality of first time periods, first mean heart-rate parameters respectively corresponding to the plurality of first time periods;
determining (S120), based on the first mean heart-rate parameters respectively corresponding to the plurality of first time periods, the first static heart-rate parameter, wherein the first static heart-rate parameter comprises a sleep rest heart rate parameter;
determining (S130) a first time period corresponding to the first static heart-rate parameter as a first static time period; and
determining (S140) a heart-rate variability parameter corresponding to the first static time period as the first static heart-rate variability parameter, wherein the first static heart-rate variability parameter comprises a sleep rest heart rate variability parameter;
the second time interval comprises a plurality of second time periods, and the determining, based on a second time interval, a second parameter set corresponding to the subject to be tested comprises:
determining (S210), based on the plurality of second time periods, second mean heart-rate parameters respectively corresponding to the plurality of second time periods;
determining (S220), based on the second mean heart-rate parameters respectively corresponding to the plurality of second time periods, the second static heart-rate parameter, wherein the second static heart-rate parameter comprises a daytime-rest heart-rate parameter;
determining (S230) a second time period corresponding to the second static heart-rate parameter as a third static time period; and
determining (S240) a heart-rate variability parameter corresponding to the third static time period as the second static heart-rate variability parameter, wherein the second static heart-rate variability parameter comprises a daytime-rest heart-rate variability parameter.

2. The parameter determination method according to claim 1, wherein the determining, based on the first mean heart-rate parameters respectively corresponding to the plurality of first time periods, the first static heart-rate parameter comprises:
determining (S121) a minimum first mean heart-rate parameter in the first mean heart-rate parameters respectively corresponding to the plurality of first time periods; and
determining (S122) the minimum first mean heart-rate parameter as the first static heart-rate parameter.

3. The parameter determination method according to claim 1, the determining, based on the first mean heart-rate parameters respectively corresponding to the plurality of first time periods, the first static heart-rate parameter comprises:
selecting a preset time interval in the first time interval, wherein a length of the preset time interval is less than a length of the first time interval;
determining a minimum first mean heart-rate parameter in the first mean heart-rate parameters respectively corresponding to the first time periods;
determining the minimum first mean heart-rate parameter as the first static heart-rate parameter.

4. The parameter determination method according to claim 1, wherein before the determining, based on the plurality of first time periods, first mean heart-rate parameters respectively corresponding to the plurality of first time periods, the method further comprises:
removing (S105) an unstable time interval corresponding to each of the plurality of first time periods; wherein
the determining, based on the plurality of first time periods, first mean heart-rate parameters respectively corresponding to the plurality of first time periods comprises:
determining (S115), based on a plurality of first time periods without the unstable time interval, the first mean heart-rate parameters respectively corresponding to the plurality of first time periods.

5. The parameter determination method according to claim 1, wherein the determining, based on the second mean heart-rate parameters respectively corresponding to the plurality of second time periods, the second static heart-rate parameter comprises:
determining (S221) a minimum second mean heart-rate parameter in the second mean heart-rate parameters respectively corresponding to the plurality of second time periods; and
determining (S222) the minimum second mean heart-rate parameter as the second static heart-rate parameter.

6. The parameter determination method according to claim 1, wherein before the determining, based on the plurality of second time periods, second mean heart-rate parameters respectively corresponding to the plurality of second time periods, the method further comprises:
removing an unstable time interval corresponding to each of the plurality of second time periods; wherein
the determining, based on the plurality of second time periods, second mean heart-rate parameters respectively corresponding to the plurality of second time periods comprises:
determining, based on a plurality of second time periods without the unstable time interval, the second mean heart-rate parameters respectively corresponding to the plurality of second time periods.

7. The parameter determination method according to any one of claims 1 to 6, wherein the first time interval comprises a time interval from 22:00 of a current day to 6:00 of a next day, and the second time interval comprises a time interval from 8:00 to 20:00 of a current day.

8. The parameter determination method according to any one of claims 1 to 7, wherein after the determining, based on a second time interval, a second parameter set corresponding to the subject to be tested, the method further comprises:
determining, based on the first parameter set and the second parameter set, a level and/or a state of neurohormone corresponding to the subject to be tested.

9. A parameter determination apparatus, comprising:
a first determination module (100), configured to determine, based on a first time interval, a first parameter set corresponding to a subject to be tested, wherein the first time interval comprises a nighttime interval, and the first parameter set comprises a first heart-rate characterization parameter and a first heart-rate variability characterization parameter, the first heart-rate characterization parameter comprises a first mean heart-rate parameter and a first static heart-rate parameter, and the first heart-rate variability characterization parameter comprises a first static heart-rate variability parameter; and
a second determination module (200), configured to determine, based on a second time interval, a second parameter set corresponding to the subject to be tested, wherein the second time interval comprises a daytime interval, and the second parameter set comprises a second heart-rate characterization parameter and a second heart-rate variability characterization parameter, the second heart-rate characterization parameter comprises a second mean heart-rate parameter and a second static heart-rate parameter, and the second heart-rate variability characterization parameter comprises a second static heart-rate variability parameter;
the first time interval comprises a plurality of first time periods, the first determination module (100) comprises:
a first mean heart-rate parameter determination unit (110), configured to determine, based on the plurality of first time periods, first mean heart-rate parameters respectively corresponding to the plurality of first time periods;
a first static heart-rate parameter determination unit (120), configured to determine, based on the first mean heart-rate parameters respectively corresponding to the plurality of first time periods, the first static heart-rate parameter, wherein the first static heart-rate parameter comprises a sleep rest heart rate parameter;
a first static time period determination unit (130), configured to determine a first time period corresponding to the first static heart-rate parameter as a first static time period; and
a first static heart-rate variability parameter determination unit (140), configured to determine a heart-rate variability parameter corresponding to the first static time period as the first static heart-rate variability parameter, wherein the first static heart-rate variability parameter comprises a sleep rest heart rate variability parameter;
the second time interval comprises a plurality of second time periods, and second determination module (200) comprises:
a second mean heart-rate parameter determination unit (210), configured to determine, based on the plurality of second time periods, second mean heart-rate parameters respectively corresponding to the plurality of second time periods;
a second static heart-rate parameter determination unit (220), configured to determine, based on the second mean heart-rate parameters respectively corresponding to the plurality of second time periods, the second static heart-rate parameter, wherein the second static heart-rate parameter comprises a daytime-rest heart-rate parameter;
a third static time period determination unit (230), configured to determine a second time period corresponding to the second static heart-rate parameter as a third static time period; and
a second static heart-rate variability parameter determination unit (240), configured to determine a heart-rate variability parameter corresponding to the third static time period as the second static heart-rate variability parameter, wherein the second static heart-rate variability parameter comprises a daytime-rest heart-rate variability parameter.

10. The parameter determination apparatus according to claim 9, further comprising:
an unstable time interval removal unit (105), configured to remove an unstable time interval corresponding to each of the plurality of first time periods; and
a first mean heart-rate parameter determination sub-unit (115), configured to determine, based on a plurality of first time periods without the unstable time interval, the first mean heart-rate parameters respectively corresponding to the plurality of first time periods.

11. The parameter determination apparatus according to claim 9 or 10, wherein the first time interval comprises a time interval from 22:00 of a current day to 6:00 of a next day, and the second time interval comprises a time interval from 8:00 to 20:00 of a current day; and
the parameter determination apparatus is further configured to:
determine, based on the first parameter set and the second parameter set, a level and/or a state of neurohormone corresponding to the subject to be tested.

12. A non-transitory computer-readable storage medium, wherein a computer program is stored for performing the parameter determination method according to any one of claims 1 to 8.

13. An electronic device, comprising:
a processor (1701); and
a memory (1702), configured to store executable instructions of the processor, wherein
the processor is configured to implement the parameter determination method according to any one of claims 1 to 8.

14. A parameter determination method, implemented by a server or a medical device, comprising:
determining (S100), based on a first time interval, a first parameter set corresponding to a subject to be tested, wherein the first time interval comprises a nighttime interval, the first parameter set comprises a first heart-rate characterization parameter and a first heart-rate variability characterization parameter, the first heart-rate characterization parameter comprises a first mean heart-rate parameter and a first static heart-rate parameter, and the first heart-rate variability characterization parameter comprises a first static heart-rate variability parameter; and
determining (S200), based on a second time interval, a second parameter set corresponding to the subject to be tested, wherein the second time interval comprises a daytime interval, the second parameter set comprises a second heart-rate characterization parameter and a second heart-rate variability characterization parameter, the second heart-rate characterization parameter comprises a second mean heart-rate parameter and a second static heart-rate parameter, and the second heart-rate variability characterization parameter comprises a second static heart-rate variability parameter;
the first time interval comprises a plurality of first time periods, the determining, based on a first time interval, a first parameter set corresponding to a subject to be tested comprises:
determining, based on the plurality of first time periods, first heart-rate variability parameters respectively corresponding to the plurality of first time periods;
determining, based on the first heart-rate variability parameters respectively corresponding to the plurality of first time periods, the first static heart-rate variability parameter, wherein the first static heart-rate variability parameter comprises a sleep rest heart rate variability parameter;
determining a first time period corresponding to the first static heart-rate variability parameter as a first static time period; and
determining a first mean heart-rate parameter corresponding to the first static time period as the first static mean heart-rate parameter, wherein the first static mean heart-rate parameter comprises a sleep rest heart rate parameter;
the second time interval comprises a plurality of second time periods, and the determining, based on a second time interval, a second parameter set corresponding to the subject to be tested comprises:
determining, based on the plurality of second time periods, second heart-rate variability parameters respectively corresponding to the plurality of second time periods;
determining, based on the second heart-rate variability parameters respectively corresponding to the plurality of second time periods, the second static heart-rate variability parameter, wherein the second static heart-rate variability parameter comprises a daytime-rest heart-rate variability parameter;
determining a second time period corresponding to the second static heart-rate variability parameter as a third static time period; and
determining a second mean heart-rate parameter corresponding to the third static time period as the second static heart-rate parameter, wherein the second static heart-rate parameter comprises a daytime-rest heart-rate parameter.

15. The parameter determination method according to claim 14, wherein the determining, based on the first heart-rate variability parameters respectively corresponding to the plurality of first time periods, the first static heart-rate variability parameter comprises:
determining a maximum first heart-rate variability parameter in the first heart-rate variability parameters respectively corresponding to the plurality of first time periods; and
determining the maximum first heart-rate variability parameter as the first static heart-rate variability parameter;
and the determining, based on the second heart-rate variability parameters respectively corresponding to the plurality of second time periods, the second static heart-rate variability parameter comprises:
determining a maximum second heart-rate variability parameter in the second heart-rate variability parameters respectively corresponding to the plurality of second time periods; and
determining the maximum second heart-rate variability parameter as the second static heart-rate variability parameter.

## Patentansprüche

1. Parameterbestimmungsverfahren, das von einem Server oder einer medizinischen Vorrichtung implementiert wird, das Folgendes umfasst:
Bestimmen (S100), basierend auf einem ersten Zeitintervall, eines ersten Parametersatzes, der einer zu testenden Subjekt entspricht, wobei das erste Zeitintervall ein Nachtintervall umfasst, der erste Parametersatz einen ersten Herzfrequenz-Charakterisierungsparameter und einen ersten Herzfrequenzvariabilitäts-Charakterisierungsparameter umfasst, der erste Herzfrequenz-Charakterisierungsparameter einen ersten mittleren Herzfrequenzparameter und einen ersten statischen Herzfrequenzparameter umfasst, und der erste Herzfrequenzvariabilitäts-Charakterisierungsparameter einen ersten statischen Herzfrequenzvariabilitätsparameter umfasst; und
Bestimmen (S200), basierend auf einem zweiten Zeitintervall, eines zweiten Parametersatzes, der dem zu testenden Subjekt entspricht, wobei das zweite Zeitintervall ein Tagesintervall umfasst, der zweite Parametersatz einen zweiten Herzfrequenz-Charakterisierungsparameter und einen zweiten Herzfrequenzvariabilitäts-Charakterisierungsparameter umfasst, der zweite Herzfrequenz-Charakterisierungsparameter einen zweiten mittleren Herzfrequenzparameter und einen zweiten statischen Herzfrequenzparameter umfasst, und der zweite Herzfrequenzvariabilitäts-Charakterisierungsparameter einen zweiten statischen Herzfrequenzvariabilitätsparameter umfasst;
das erste Zeitintervall eine Vielzahl von ersten Zeiträumen umfasst, das Bestimmen, basierend auf einem ersten Zeitintervall, eines ersten Parametersatzes, der einer zu testenden Subjekt entspricht, Folgendes umfasst:
Bestimmen (S110), basierend auf der Vielzahl von ersten Zeiträumen, von ersten mittleren Herzfrequenzparametern, die jeweils der Vielzahl von ersten Zeiträumen entsprechen;
Bestimmen (S120), basierend auf den ersten mittleren Herzfrequenzparametern, die jeweils der Vielzahl von ersten Zeiträumen entsprechen, des ersten statischen Herzfrequenzparameters, wobei der erste statische Herzfrequenzparameter einen Schlaf-Ruhe-Herzfrequenzparameter umfasst;
Bestimmen (S130) eines ersten Zeitraums, der dem ersten statischen Herzfrequenzparameter entspricht, als einen ersten statischen Zeitraum; und
Bestimmen (S140) eines Herzfrequenzvariabilitätsparameters, der dem ersten statischen Zeitraum entspricht, als den ersten statischen Herzfrequenzvariabilitätsparameter, wobei der erste statische Herzfrequenzvariabilitätsparameter einen Schlaf-Ruhe-Herzfrequenzvariabilitätsparameter umfasst;
das zweite Zeitintervall eine Vielzahl von zweiten Zeiträumen umfasst und das Bestimmen, basierend auf einem zweiten Zeitintervall, eines zweiten Parametersatzes, der dem zu testenden Subjekt entspricht, Folgendes umfasst:
Bestimmen (S210), basierend auf der Vielzahl von zweiten Zeiträumen, von zweiten mittleren Herzfrequenzparametern, die jeweils der Vielzahl von zweiten Zeiträumen entsprechen;
Bestimmen (S220), basierend auf den zweiten mittleren Herzfrequenzparametern, die jeweils der Vielzahl von zweiten Zeiträumen entsprechen, des zweiten statischen Herzfrequenzparameters, wobei der zweite statische Herzfrequenzparameter einen Tag-Ruhe-Herzfrequenzparameter umfasst;
Bestimmen (S230) eines zweiten Zeitraums, der dem zweiten statischen Herzfrequenzparameter entspricht, als einen dritten statischen Zeitraum; und
Bestimmen (S240) eines Herzfrequenzvariabilitätsparameters, der dem dritten statischen Zeitraum entspricht, als den zweiten statischen Herzfrequenzvariabilitätsparameter, wobei der zweite statische Herzfrequenzvariabilitätsparameter einen Tag-Ruhe-Herzfrequenzvariabilitätsparameter umfasst.

2. Parameterbestimmungsverfahren nach Anspruch 1, wobei das Bestimmen, basierend auf den ersten mittleren Herzfrequenzparametern, die jeweils der Vielzahl von ersten Zeiträumen entsprechen, des ersten statischen Herzfrequenzparameters Folgendes umfasst:
Bestimmen (S121) eines minimalen ersten mittleren Herzfrequenzparameters in den ersten mittleren Herzfrequenzparametern, die jeweils der Vielzahl von ersten Zeiträumen entsprechen; und
Bestimmen (S122) des minimalen ersten mittleren Herzfrequenzparameters als den ersten statischen Herzfrequenzparameter.

3. Parameterbestimmungsverfahren nach Anspruch 1, wobei das Bestimmen, basierend auf den ersten mittleren Herzfrequenzparametern, die jeweils der Vielzahl von ersten Zeiträumen entsprechen, des ersten statischen Herzfrequenzparameters Folgendes umfasst:
Auswählen eines voreingestellten Zeitintervalls in dem ersten Zeitintervall, wobei eine Länge des voreingestellten Zeitintervalls weniger beträgt als eine Länge des ersten Zeitintervalls;
Bestimmen eines minimalen ersten mittleren Herzfrequenzparameters in den ersten mittleren Herzfrequenzparametern, die jeweils den ersten Zeiträumen entsprechen;
Bestimmen des minimalen ersten mittleren Herzfrequenzparameters als den ersten statischen Herzfrequenzparameter.

4. Parameterbestimmungsverfahren nach Anspruch 1, wobei vor dem Bestimmen, basierend auf der Vielzahl von ersten Zeiträumen, von ersten mittleren Herzfrequenzparametern, die jeweils der Vielzahl von ersten Zeiträumen entsprechen, das Verfahren ferner Folgendes umfasst:
Entfernen (S105) eines instabilen Zeitintervalls, das jedem der Vielzahl von ersten Zeiträumen entspricht; wobei
das Bestimmen, basierend auf der Vielzahl von ersten Zeiträumen, von ersten mittleren Herzfrequenzparametern, die jeweils der Vielzahl von ersten Zeiträumen entsprechen, Folgendes umfasst:
Bestimmen (S115), basierend auf einer Vielzahl von ersten Zeiträumen ohne das instabile Zeitintervall, der ersten mittleren Herzfrequenzparameter, die jeweils der Vielzahl von ersten Zeiträumen entsprechen.

5. Parameterbestimmungsverfahren nach Anspruch 1, wobei das Bestimmen, basierend auf den zweiten mittleren Herzfrequenzparametern, die jeweils der Vielzahl von zweiten Zeiträumen entsprechen, des zweiten statischen Herzfrequenzparameters Folgendes umfasst:
Bestimmen (S221) eines minimalen zweiten mittleren Herzfrequenzparameters in den zweiten mittleren Herzfrequenzparametern, die jeweils der Vielzahl von zweiten Zeiträumen entsprechen; und
Bestimmen (S222) des minimalen zweiten mittleren Herzfrequenzparameters als den zweiten statischen Herzfrequenzparameter.

6. Parameterbestimmungsverfahren nach Anspruch 1, wobei vor dem Bestimmen, basierend auf der Vielzahl von zweiten Zeiträumen, von zweiten mittleren Herzfrequenzparametern, die jeweils der Vielzahl von zweiten Zeiträumen entsprechen, das Verfahren ferner Folgendes umfasst:
Entfernen eines instabilen Zeitintervalls, das jedem der Vielzahl von zweiten Zeiträumen entspricht; wobei
das Bestimmen, basierend auf der Vielzahl von zweiten Zeiträumen, von zweiten mittleren Herzfrequenzparametern, die j eweils der Vielzahl von zweiten Zeiträumen entsprechen, Folgendes umfasst:
Bestimmen, basierend auf einer Vielzahl von zweiten Zeiträumen ohne das instabile Zeitintervall, der zweiten mittleren Herzfrequenzparameter, die jeweils der Vielzahl von zweiten Zeiträumen entsprechen.

7. Parameterbestimmungsverfahren nach einem der Ansprüche 1 bis 6, wobei das erste Zeitintervall ein Zeitintervall von 22:00 Uhr eines aktuellen Tages bis 6:00 Uhr eines nächsten Tages umfasst und das zweite Zeitintervall ein Zeitintervall von 8:00 Uhr bis 20:00 Uhr eines aktuellen Tages umfasst.

8. Parameterbestimmungsverfahren nach einem der Ansprüche 1 bis 7, wobei das Verfahren nach dem Bestimmen, basierend auf einem zweiten Zeitintervall, eines zweiten Parametersatzes, der dem zu testenden Subjekt entspricht, ferner Folgendes umfasst:
Bestimmen, basierend auf dem ersten Parametersatz und dem zweiten Parametersatz, eines Niveaus und/oder eines Zustands eines Neurohormons, das dem zu testenden Subjekt entspricht.

9. Parameterbestimmungseinrichtung, die Folgendes umfasst:
ein erstes Bestimmungsmodul (100), das konfiguriert ist, um basierend auf einem ersten Zeitintervall einen ersten Parametersatz zu bestimmen, der einer zu testenden Subjekt entspricht, wobei das erste Zeitintervall ein Nachtintervall umfasst und der erste Parametersatz einen ersten Herzfrequenz-Charakterisierungsparameter und einen ersten Herzfrequenzvariabilitäts-Charakterisierungsparameter umfasst, der erste Herzfrequenz-Charakterisierungsparameter einen ersten mittleren Herzfrequenzparameter und einen ersten statischen Herzfrequenzparameter umfasst, und der erste Herzfrequenzvariabilitäts-Charakterisierungsparameter einen ersten statischen Herzfrequenzvariabilitätsparameter umfasst; und
ein zweites Bestimmungsmodul (200), das konfiguriert ist, um basierend auf einem zweiten Zeitintervall einen zweiten Parametersatz zu bestimmen, der dem zu testenden Subjekt entspricht, wobei das zweite Zeitintervall ein Tagesintervall umfasst und der zweite Parametersatz einen zweiten Herzfrequenz-Charakterisierungsparameter und einen zweiten Herzfrequenzvariabilitäts-Charakterisierungsparameter umfasst, der zweite Herzfrequenz-Charakterisierungsparameter einen zweiten mittleren Herzfrequenzparameter und einen zweiten statischen Herzfrequenzparameter umfasst, und der zweite Herzfrequenzvariabilitäts-Charakterisierungsparameter einen zweiten statischen Herzfrequenzvariabilitätsparameter umfasst;
das erste Zeitintervall eine Vielzahl von ersten Zeiträumen umfasst, das erste Bestimmungsmodul (100) Folgendes umfasst:
eine Bestimmungseinheit (110) des ersten mittleren Herzfrequenzparameters, die konfiguriert ist, um basierend auf der Vielzahl von ersten Zeiträumen erste mittlere Herzfrequenzparameter zu bestimmen, die jeweils der Vielzahl von ersten Zeiträumen entsprechen;
eine Bestimmungseinheit (120) des ersten statischen Herzfrequenzparameters, die konfiguriert ist, um basierend auf den ersten mittleren Herzfrequenzparametern, die jeweils der Vielzahl von ersten Zeiträumen entsprechen, den ersten statischen Herzfrequenzparameter zu bestimmen, wobei der erste statische Herzfrequenzparameter einen Schlaf-Ruhe-Herzfrequenzparameter umfasst;
eine Bestimmungseinheit (130) des ersten statischen Zeitraums, die konfiguriert ist, um einen ersten Zeitraum, der dem ersten statischen Herzfrequenzparameter entspricht, als einen ersten statischen Zeitraum zu bestimmen; und
eine Bestimmungseinheit (140) des ersten statischen Herzfrequenzvariabilitätsparameters, die konfiguriert ist, um einen Herzfrequenzvariabilitätsparameter, der dem ersten statischen Zeitraum entspricht, als den ersten statischen Herzfrequenzvariabilitätsparameter zu bestimmen, wobei der erste statische Herzfrequenzvariabilitätsparameter einen Schlaf-Ruhe-Herzfrequenzvariabilitätsparameter umfasst;
das zweite Zeitintervall eine Vielzahl von zweiten Zeiträumen umfasst und das zweite Bestimmungsmodul (200) Folgendes umfasst:
eine Bestimmungseinheit (210) des zweiten mittleren Herzfrequenzparameters, die konfiguriert ist, um basierend auf der Vielzahl von zweiten Zeiträumen zweite mittlere Herzfrequenzparameter zu bestimmen, die jeweils der Vielzahl von zweiten Zeiträumen entsprechen;
eine Bestimmungseinheit (220) des zweiten statischen Herzfrequenzparameters, die konfiguriert ist, um basierend auf den zweiten mittleren Herzfrequenzparametern, die jeweils der Vielzahl von zweiten Zeiträumen entsprechen, den zweiten statischen Herzfrequenzparameter zu bestimmen, wobei der zweite statische Herzfrequenzparameter einen Tag-Ruhe-Herzfrequenzparameter umfasst;
eine Bestimmungseinheit (230) des dritten statischen Zeitraums, die konfiguriert ist, um einen zweiten Zeitraum, der dem zweiten statischen Herzfrequenzparameter entspricht, als einen dritten statischen Zeitraum zu bestimmen; und
eine Bestimmungseinheit (240) des zweiten statischen Herzfrequenzvariabilitätsparameters, die konfiguriert ist, um einen Herzfrequenzvariabilitätsparameter, der dem dritten statischen Zeitraum entspricht, als den zweiten statischen Herzfrequenzvariabilitätsparameter zu bestimmen, wobei der zweite statische Herzfrequenzvariabilitätsparameter einen Tag-Ruhe-Herzfrequenzvariabilitätsparameter umfasst.

10. Parameterbestimmungseinrichtung nach Anspruch 9, ferner umfassend:
eine Einheit (105) zum Entfernen instabiler Zeitintervalle, die konfiguriert ist, um ein instabiles Zeitintervall zu entfernen, das jedem der Vielzahl von ersten Zeiträumen entspricht; und
eine Untereinheit (115) zur Bestimmung des ersten mittleren Herzfrequenzparameters, die konfiguriert ist, um basierend auf einer Vielzahl von ersten Zeiträumen ohne das instabile Zeitintervall die ersten mittleren Herzfrequenzparameter zu bestimmen, die jeweils der Vielzahl von ersten Zeiträumen entsprechen.

11. Parameterbestimmungseinrichtung nach Anspruch 9 oder 10, wobei das erste Zeitintervall ein Zeitintervall von 22:00 Uhr eines aktuellen Tages bis 6:00 Uhr eines nächsten Tages umfasst und das zweite Zeitintervall ein Zeitintervall von 8:00 Uhr bis 20:00 Uhr eines aktuellen Tages umfasst; und
die Parameterbestimmungseinrichtung ferner konfiguriert ist zum:
Bestimmen, basierend auf dem ersten Parametersatz und dem zweiten Parametersatz, eines Niveaus und/oder eines Zustands eines Neurohormons, das dem zu testenden Subjekt entspricht.

12. Nichtflüchtiges computerlesbares Speichermedium, wobei ein Computerprogramm zum Durchführen des Parameterbestimmungsverfahrens nach einem der Ansprüche 1 bis 8 gespeichert ist.

13. Elektronische Vorrichtung, umfassend:
einen Prozessor (1701); und
einen Speicher (1702), der zum Speichern ausführbarer Anweisungen des Prozessors konfiguriert ist, wobei
der Prozessor konfiguriert ist, um das Parameterbestimmungsverfahren nach einem der Ansprüche 1 bis 8 zu implementieren.

14. Parameterbestimmungsverfahren, das von einem Server oder einer medizinischen Vorrichtung implementiert wird, das Folgendes umfasst:
Bestimmen (S100), basierend auf einem ersten Zeitintervall, eines ersten Parametersatzes, der einer zu testenden Subjekt entspricht, wobei das erste Zeitintervall ein Nachtintervall umfasst, der erste Parametersatz einen ersten Herzfrequenz-Charakterisierungsparameter und einen ersten Herzfrequenzvariabilitäts-Charakterisierungsparameter umfasst, der erste Herzfrequenz-Charakterisierungsparameter einen ersten mittleren Herzfrequenzparameter und einen ersten statischen Herzfrequenzparameter umfasst, und der erste Herzfrequenzvariabilitäts-Charakterisierungsparameter einen ersten statischen Herzfrequenzvariabilitätsparameter umfasst; und
Bestimmen (S200), basierend auf einem zweiten Zeitintervall, eines zweiten Parametersatzes, der dem zu testenden Subjekt entspricht, wobei das zweite Zeitintervall ein Tagesintervall umfasst, der zweite Parametersatz einen zweiten Herzfrequenz-Charakterisierungsparameter und einen zweiten Herzfrequenzvariabilitäts-Charakterisierungsparameter umfasst, der zweite Herzfrequenz-Charakterisierungsparameter einen zweiten mittleren Herzfrequenzparameter und einen zweiten statischen Herzfrequenzparameter umfasst, und der zweite Herzfrequenzvariabilitäts-Charakterisierungsparameter einen zweiten statischen Herzfrequenzvariabilitätsparameter umfasst;
das erste Zeitintervall eine Vielzahl von ersten Zeiträumen umfasst, das Bestimmen, basierend auf einem ersten Zeitintervall, eines ersten Parametersatzes, der einer zu testenden Subjekt entspricht, Folgendes umfasst:
Bestimmen, basierend auf der Vielzahl von ersten Zeiträumen, von ersten Herzfrequenzvariabilitätsparametern, die jeweils der Vielzahl von ersten Zeiträumen entsprechen;
Bestimmen, basierend auf den ersten Herzfrequenzvariabilitätsparametern, die jeweils der Vielzahl von ersten Zeiträumen entsprechen, des ersten statischen Herzfrequenzvariabilitätsparameters, wobei der erste statische Herzfrequenzvariabilitätsparameter einen Schlaf-Ruhe-Herzfrequenzvariabilitätsparameter umfasst;
Bestimmen eines ersten Zeitraums, der dem ersten statischen Herzfrequenzvariabilitätsparameter entspricht, als einen ersten statischen Zeitraum; und
Bestimmen eines ersten mittleren Herzfrequenzparameters, der dem ersten statischen Zeitraum entspricht, als den ersten statischen mittleren Herzfrequenzparameter, wobei der erste statische mittlere Herzfrequenzparameter einen Schlaf-Ruhe-Herzfrequenzparameter umfasst;
das zweite Zeitintervall eine Vielzahl von zweiten Zeiträumen umfasst und das Bestimmen, basierend auf einem zweiten Zeitintervall, eines zweiten Parametersatzes, der dem zu testenden Subjekt entspricht, Folgendes umfasst:
Bestimmen, basierend auf der Vielzahl von zweiten Zeiträumen, von zweiten Herzfrequenzvariabilitätsparametern, die jeweils der Vielzahl von zweiten Zeiträumen entsprechen;
Bestimmen, basierend auf den zweiten Herzfrequenzvariabilitätsparametern, die jeweils der Vielzahl von zweiten Zeiträumen entsprechen, des zweiten statischen Herzfrequenzvariabilitätsparameters, wobei der zweite statische Herzfrequenzvariabilitätsparameter einen Tag-Ruhe-Herzfrequenzvariabilitätsparameter umfasst;
Bestimmen eines zweiten Zeitraums, der dem zweiten statischen Herzfrequenzvariabilitätsparameter entspricht, als einen dritten statischen Zeitraum; und
Bestimmen eines zweiten mittleren Herzfrequenzparameters, der dem dritten statischen Zeitraum entspricht, als den zweiten statischen Herzfrequenzparameter, wobei der zweite statische Herzfrequenzparameter einen Tag-Ruhe-Herzfrequenzparameter umfasst.

15. Parameterbestimmungsverfahren nach Anspruch 14, wobei das Bestimmen, basierend auf den ersten Herzfrequenzvariabilitätsparametern, die jeweils der Vielzahl von ersten Zeiträumen entsprechen, des ersten statischen Herzfrequenzvariabilitätsparameters Folgendes umfasst:
Bestimmen eines maximalen ersten Herzfrequenzvariabilitätsparameters in den ersten Herzfrequenzvariabilitätsparametern, die jeweils der Vielzahl von ersten Zeiträumen entsprechen; und
Bestimmen des maximalen ersten Herzfrequenzvariabilitätsparameters als den ersten statischen Herzfrequenzvariabilitätsparameter;
und das Bestimmen, basierend auf den zweiten Herzfrequenzvariabilitätsparametern, die jeweils der Vielzahl von zweiten Zeiträumen entsprechen, des zweiten statischen Herzfrequenzvariabilitätsparameters Folgendes umfasst:
Bestimmen eines maximalen zweiten Herzfrequenzvariabilitätsparameters in den zweiten Herzfrequenzvariabilitätsparametern, die jeweils der Vielzahl von zweiten Zeiträumen entsprechen; und
Bestimmen des maximalen zweiten Herzfrequenzvariabilitätsparameters als den zweiten statischen Herzfrequenzvariabilitätsparameter.

## Revendications

1. Procédé de détermination de paramètres, mis en œuvre par un serveur ou un dispositif médical, comprenant :
la détermination (S100), sur la base d'un premier intervalle de temps, d'un premier ensemble de paramètres correspondant à un sujet à tester, dans laquelle le premier intervalle de temps comprend un intervalle nocturne, le premier ensemble de paramètres comprend un premier paramètre de caractérisation de fréquence cardiaque et un premier paramètre de caractérisation de variabilité de fréquence cardiaque, le premier paramètre de caractérisation de fréquence cardiaque comprend un premier paramètre de fréquence cardiaque moyen et un premier paramètre de fréquence cardiaque statique, et le premier paramètre de caractérisation de variabilité de fréquence cardiaque comprend un premier paramètre de variabilité de fréquence cardiaque statique ; et
la détermination (S200), sur la base d'un deuxième intervalle de temps, d'un deuxième ensemble de paramètres correspondant au sujet à tester, dans laquelle le deuxième intervalle de temps comprend un intervalle diurne, le deuxième ensemble de paramètres comprend un deuxième paramètre de caractérisation de fréquence cardiaque et un deuxième paramètre de caractérisation de variabilité de fréquence cardiaque, le deuxième paramètre de caractérisation de fréquence cardiaque comprend un deuxième paramètre de fréquence cardiaque moyen et un deuxième paramètre de fréquence cardiaque statique, et le deuxième paramètre de caractérisation de variabilité de fréquence cardiaque comprend un deuxième paramètre de variabilité de fréquence cardiaque statique ;
le premier intervalle de temps comprend une pluralité de premières périodes de temps, la détermination, sur la base d'un premier intervalle de temps, d'un premier ensemble de paramètres correspondant à un sujet à tester comprend :
la détermination (S110), sur la base de la pluralité de premières périodes de temps, de premiers paramètres de fréquence cardiaque moyens correspondant respectivement à la pluralité de premières périodes de temps ;
la détermination (S120), sur la base des premiers paramètres de fréquence cardiaque moyens correspondant respectivement à la pluralité de premières périodes de temps, du premier paramètre de fréquence cardiaque statique, dans laquelle le premier paramètre de fréquence cardiaque statique comprend un paramètre de fréquence cardiaque de repos en sommeil ;
la détermination (S130) d'une première période de temps correspondant au premier paramètre de fréquence cardiaque statique en tant que première période de temps statique ; et
la détermination (S140) d'un paramètre de variabilité de fréquence cardiaque correspondant à la première période de temps statique en tant que premier paramètre de variabilité de fréquence cardiaque statique, dans laquelle le premier paramètre de variabilité de fréquence cardiaque statique comprend un paramètre de variabilité de fréquence cardiaque de repos en sommeil ;
le deuxième intervalle de temps comprend une pluralité de deuxièmes périodes de temps, et la détermination, sur la base d'un deuxième intervalle de temps, d'un deuxième ensemble de paramètres correspondant au sujet à tester comprend :
la détermination (S210), sur la base de la pluralité de deuxièmes périodes de temps, de deuxièmes paramètres de fréquence cardiaque moyens correspondant respectivement à la pluralité de deuxièmes périodes de temps ;
la détermination (S220), sur la base des deuxièmes paramètres de fréquence cardiaque moyens correspondant respectivement à la pluralité de deuxièmes périodes de temps, du deuxième paramètre de fréquence cardiaque statique, dans laquelle le deuxième paramètre de fréquence cardiaque statique comprend un paramètre de fréquence cardiaque en repos diurne ;
la détermination (S230) d'une deuxième période de temps correspondant au deuxième paramètre de fréquence cardiaque statique en tant que troisième période de temps statique ; et
la détermination (S240) d'un paramètre de variabilité de fréquence cardiaque correspondant à la troisième période de temps statique en tant que deuxième paramètre de variabilité de fréquence cardiaque statique, dans laquelle le deuxième paramètre de variabilité de fréquence cardiaque statique comprend un paramètre de variabilité de fréquence cardiaque en repos diurne.

2. Procédé de détermination de paramètres selon la revendication 1, dans lequel la détermination, sur la base des premiers paramètres de fréquence cardiaque moyens correspondant respectivement à la pluralité de premières périodes de temps, du premier paramètre de fréquence cardiaque statique comprend :
la détermination (S121) d'un premier paramètre de fréquence cardiaque moyen minimal dans les premiers paramètres de fréquence cardiaque moyens correspondant respectivement à la pluralité de premières périodes de temps ; et
la détermination (S122) du premier paramètre de fréquence cardiaque moyen minimal en tant que premier paramètre de fréquence cardiaque statique.

3. Procédé de détermination de paramètres selon la revendication 1, dans lequel la détermination, sur la base des premiers paramètres de fréquence cardiaque moyens correspondant respectivement à la pluralité de premières périodes de temps, du premier paramètre de fréquence cardiaque statique comprend :
la sélection d'un intervalle de temps prédéfini dans le premier intervalle de temps, dans laquelle une longueur de l'intervalle de temps prédéfini est inférieure à une longueur du premier intervalle de temps ;
la détermination d'un premier paramètre de fréquence cardiaque moyen minimal dans les premiers paramètres de fréquence cardiaque moyens correspondant respectivement aux premières périodes de temps ;
la détermination du premier paramètre de fréquence cardiaque moyen minimal en tant que premier paramètre de fréquence cardiaque statique.

4. Procédé de détermination de paramètres selon la revendication 1, dans lequel avant la détermination, sur la base de la pluralité de premières périodes de temps, de premiers paramètres de fréquence cardiaque moyens correspondant respectivement à la pluralité de premières périodes de temps, le procédé comprend en outre :
la suppression (S105) d'un intervalle de temps instable correspondant à chacune de la pluralité de premières périodes de temps ; dans lequel
la détermination, sur la base de la pluralité de premières périodes de temps, de premiers paramètres de fréquence cardiaque moyens correspondant respectivement à la pluralité de premières périodes de temps comprend :
la détermination (S115), sur la base d'une pluralité de premières périodes de temps sans l'intervalle de temps instable, des premiers paramètres de fréquence cardiaque moyens correspondant respectivement à la pluralité de premières périodes de temps.

5. Procédé de détermination de paramètres selon la revendication 1, dans lequel la détermination, sur la base des deuxièmes paramètres de fréquence cardiaque moyens correspondant respectivement à la pluralité de deuxièmes périodes de temps, du deuxième paramètre de fréquence cardiaque statique comprend :
la détermination (S221) d'un deuxième paramètre de fréquence cardiaque moyen minimal dans les deuxièmes paramètres de fréquence cardiaque moyens correspondant respectivement à la pluralité de deuxièmes périodes de temps ; et
la détermination (S222) du deuxième paramètre de fréquence cardiaque moyen minimal en tant que deuxième paramètre de fréquence cardiaque statique.

6. Procédé de détermination de paramètres selon la revendication 1, dans lequel avant la détermination, sur la base de la pluralité de deuxièmes périodes de temps, de deuxièmes paramètres de fréquence cardiaque moyens correspondant respectivement à la pluralité de deuxièmes périodes de temps, le procédé comprend en outre :
la suppression d'un intervalle de temps instable correspondant à chacune de la pluralité de deuxièmes périodes de temps ; dans lequel
la détermination, sur la base de la pluralité de deuxièmes périodes de temps, de deuxièmes paramètres de fréquence cardiaque moyens correspondant respectivement à la pluralité de deuxièmes périodes de temps comprend :
la détermination, sur la base d'une pluralité de deuxièmes périodes de temps sans l'intervalle de temps instable, des deuxièmes paramètres de fréquence cardiaque moyens correspondant respectivement à la pluralité de deuxièmes périodes de temps.

7. Procédé de détermination de paramètres selon l'une quelconque des revendications 1 à 6, dans lequel le premier intervalle de temps comprend un intervalle de temps de 22h00 d'un jour en cours à 6h00 d'un jour suivant, et le deuxième intervalle de temps comprend un intervalle de temps de 8h00 à 20h00 d'un jour en cours.

8. Procédé de détermination de paramètres selon l'une quelconque des revendications 1 à 7, dans lequel après la détermination, sur la base d'un deuxième intervalle de temps, d'un deuxième ensemble de paramètres correspondant au sujet à tester, le procédé comprend en outre :
la détermination, sur la base du premier ensemble de paramètres et du deuxième ensemble de paramètres, d'un niveau et/ou d'un état de neurohormone correspondant au sujet à tester.

9. Appareil de détermination de paramètres, comprenant :
un premier module de détermination (100), configuré pour déterminer, sur la base d'un premier intervalle de temps, un premier ensemble de paramètres correspondant à un sujet à tester, dans lequel le premier intervalle de temps comprend un intervalle nocturne, et le premier ensemble de paramètres comprend un premier paramètre de caractérisation de fréquence cardiaque et un premier paramètre de caractérisation de variabilité de fréquence cardiaque, le premier paramètre de caractérisation de fréquence cardiaque comprend un premier paramètre de fréquence cardiaque moyen et un premier paramètre de fréquence cardiaque statique, et le premier paramètre de caractérisation de variabilité de fréquence cardiaque comprend un premier paramètre de variabilité de fréquence cardiaque statique ; et
un deuxième module de détermination (200), configuré pour déterminer, sur la base d'un deuxième intervalle de temps, un deuxième ensemble de paramètres correspondant au sujet à tester, dans lequel le deuxième intervalle de temps comprend un intervalle diurne, et le deuxième ensemble de paramètres comprend un deuxième paramètre de caractérisation de fréquence cardiaque et un deuxième paramètre de caractérisation de variabilité de fréquence cardiaque, le deuxième paramètre de caractérisation de fréquence cardiaque comprend un deuxième paramètre de fréquence cardiaque moyen et un deuxième paramètre de fréquence cardiaque statique, et le deuxième paramètre de caractérisation de variabilité de fréquence cardiaque comprend un deuxième paramètre de variabilité de fréquence cardiaque statique ;
le premier intervalle de temps comprend une pluralité de premières périodes de temps, le premier module de détermination (100) comprend :
une première unité de détermination de paramètre de fréquence cardiaque moyen (110), configurée pour déterminer, sur la base de la pluralité de premières périodes de temps, des premiers paramètres de fréquence cardiaque moyens correspondant respectivement à la pluralité de premières périodes de temps ;
une première unité de détermination de paramètre de fréquence cardiaque statique (120), configurée pour déterminer, sur la base des premiers paramètres de fréquence cardiaque moyens correspondant respectivement à la pluralité de premières périodes de temps, le premier paramètre de fréquence cardiaque statique, dans lequel le premier paramètre de fréquence cardiaque statique comprend un paramètre de fréquence cardiaque de repos en sommeil ;
une première unité de détermination de période de temps statique (130), configurée pour déterminer une première période de temps correspondant au premier paramètre de fréquence cardiaque statique en tant que première période de temps statique ; et
une première unité de détermination de paramètre de variabilité de fréquence cardiaque statique (140), configurée pour déterminer un paramètre de variabilité de fréquence cardiaque correspondant à la première période de temps statique en tant que premier paramètre de variabilité de fréquence cardiaque statique, dans lequel le premier paramètre de variabilité de fréquence cardiaque statique comprend un paramètre de variabilité de fréquence cardiaque de repos en sommeil ;
le deuxième intervalle de temps comprend une pluralité de deuxièmes périodes de temps, et le deuxième module de détermination (200) comprend :
une deuxième unité de détermination de paramètre de fréquence cardiaque moyen (210), configurée pour déterminer, sur la base de la pluralité de deuxièmes périodes de temps, des deuxièmes paramètres de fréquence cardiaque moyens correspondant respectivement à la pluralité de deuxièmes périodes de temps ;
une deuxième unité de détermination de paramètre de fréquence cardiaque statique (220), configurée pour déterminer, sur la base des deuxièmes paramètres de fréquence cardiaque moyens correspondant respectivement à la pluralité de deuxièmes périodes de temps, le deuxième paramètre de fréquence cardiaque statique, dans lequel le deuxième paramètre de fréquence cardiaque statique comprend un paramètre de fréquence cardiaque en repos diurne ;
une troisième unité de détermination de période de temps statique (230), configurée pour déterminer une deuxième période de temps correspondant au deuxième paramètre de fréquence cardiaque statique en tant que troisième période de temps statique ; et
une deuxième unité de détermination de paramètre de variabilité de fréquence cardiaque statique (240), configurée pour déterminer un paramètre de variabilité de fréquence cardiaque correspondant à la troisième période de temps statique en tant que deuxième paramètre de variabilité de fréquence cardiaque statique, dans lequel le deuxième paramètre de variabilité de fréquence cardiaque statique comprend un paramètre de variabilité de fréquence cardiaque en repos diurne.

10. Appareil de détermination de paramètres selon la revendication 9, comprenant en outre :
une unité de suppression d'intervalle de temps instable (105), configurée pour supprimer un intervalle de temps instable correspondant à chacune de la pluralité de premières périodes de temps ; et
une première sous-unité de détermination de paramètre de fréquence cardiaque moyen (115), configurée pour déterminer, sur la base d'une pluralité de premières périodes de temps sans l'intervalle de temps instable, les premiers paramètres de fréquence cardiaque moyens correspondant respectivement à la pluralité de premières périodes de temps.

11. Appareil de détermination de paramètres selon la revendication 9 ou 10, dans lequel le premier intervalle de temps comprend un intervalle de temps de 22h00 d'un jour en cours à 6h00 d'un jour suivant, et le deuxième intervalle de temps comprend un intervalle de temps de 8h00 à 20h00 d'un jour en cours ; et
l'appareil de détermination de paramètres est en outre configuré pour :
déterminer, sur la base du premier ensemble de paramètres et du deuxième ensemble de paramètres, un niveau et/ou un état de neurohormone correspondant au sujet à tester.

12. Support de stockage non transitoire lisible par ordinateur, dans lequel un programme informatique est stocké pour réaliser le procédé de détermination de paramètres selon l'une quelconque des revendications 1 à 8.

13. Dispositif électronique, comprenant :
un processeur (1701) ; et
une mémoire (1702), configurée pour stocker des instructions exécutables du processeur, dans lequel
le processeur est configuré pour mettre en œuvre le procédé de détermination de paramètres selon l'une quelconque des revendications 1 à 8.

14. Procédé de détermination de paramètres, mis en œuvre par un serveur ou un dispositif médical, comprenant :
la détermination (S100), sur la base d'un premier intervalle de temps, d'un premier ensemble de paramètres correspondant à un sujet à tester, dans laquelle le premier intervalle de temps comprend un intervalle nocturne, le premier ensemble de paramètres comprend un premier paramètre de caractérisation de fréquence cardiaque et un premier paramètre de caractérisation de variabilité de fréquence cardiaque, le premier paramètre de caractérisation de fréquence cardiaque comprend un premier paramètre de fréquence cardiaque moyen et un premier paramètre de fréquence cardiaque statique, et le premier paramètre de caractérisation de variabilité de fréquence cardiaque comprend un premier paramètre de variabilité de fréquence cardiaque statique ; et
la détermination (S200), sur la base d'un deuxième intervalle de temps, d'un deuxième ensemble de paramètres correspondant au sujet à tester, dans laquelle le deuxième intervalle de temps comprend un intervalle diurne, le deuxième ensemble de paramètres comprend un deuxième paramètre de caractérisation de fréquence cardiaque et un deuxième paramètre de caractérisation de variabilité de fréquence cardiaque, le deuxième paramètre de caractérisation de fréquence cardiaque comprend un deuxième paramètre de fréquence cardiaque moyen et un deuxième paramètre de fréquence cardiaque statique, et le deuxième paramètre de caractérisation de variabilité de fréquence cardiaque comprend un deuxième paramètre de variabilité de fréquence cardiaque statique ;
le premier intervalle de temps comprend une pluralité de premières périodes de temps, la détermination, sur la base d'un premier intervalle de temps, d'un premier ensemble de paramètres correspondant à un sujet à tester comprend :
la détermination, sur la base de la pluralité de premières périodes de temps, de premiers paramètres de variabilité de fréquence cardiaque correspondant respectivement à la pluralité de premières périodes de temps ;
la détermination, sur la base des premiers paramètres de variabilité de fréquence cardiaque correspondant respectivement à la pluralité de premières périodes de temps, du premier paramètre de variabilité de fréquence cardiaque statique, dans laquelle le premier paramètre de variabilité de fréquence cardiaque statique comprend un paramètre de variabilité de fréquence cardiaque de repos en sommeil ;
la détermination d'une première période de temps correspondant au premier paramètre de variabilité de fréquence cardiaque statique en tant que première période de temps statique ; et
la détermination d'un premier paramètre de fréquence cardiaque moyen correspondant à la première période de temps statique en tant que premier paramètre de fréquence cardiaque moyen statique, dans laquelle le premier paramètre de fréquence cardiaque moyen statique comprend un paramètre de fréquence cardiaque de repos en sommeil ;
le deuxième intervalle de temps comprend une pluralité de deuxièmes périodes de temps, et la détermination, sur la base d'un deuxième intervalle de temps, d'un deuxième ensemble de paramètres correspondant au sujet à tester comprend :
la détermination, sur la base de la pluralité de deuxièmes périodes de temps, de deuxièmes paramètres de variabilité de fréquence cardiaque correspondant respectivement à la pluralité de deuxièmes périodes de temps ;
la détermination, sur la base des deuxièmes paramètres de variabilité de fréquence cardiaque correspondant respectivement à la pluralité de deuxièmes périodes de temps, du deuxième paramètre de variabilité de fréquence cardiaque statique, dans laquelle le deuxième paramètre de variabilité de fréquence cardiaque statique comprend un paramètre de variabilité de fréquence cardiaque en repos diurne ;
la détermination d'une deuxième période de temps correspondant au deuxième paramètre de variabilité de fréquence cardiaque statique en tant que troisième période de temps statique ; et
la détermination d'un deuxième paramètre de fréquence cardiaque moyen correspondant à la troisième période de temps statique en tant que deuxième paramètre de fréquence cardiaque statique, dans laquelle le deuxième paramètre de fréquence cardiaque statique comprend un paramètre de fréquence cardiaque en repos diurne.

15. Procédé de détermination de paramètres selon la revendication 14, dans lequel la détermination, sur la base des premiers paramètres de variabilité de fréquence cardiaque correspondant respectivement à la pluralité de premières périodes de temps, du premier paramètre de variabilité de fréquence cardiaque statique comprend :
la détermination d'un premier paramètre de variabilité de fréquence cardiaque maximal dans les premiers paramètres de variabilité de fréquence cardiaque correspondant respectivement à la pluralité de premières périodes de temps ; et
la détermination du premier paramètre de variabilité de fréquence cardiaque maximal en tant que premier paramètre de variabilité de fréquence cardiaque statique ;
et la détermination, sur la base des deuxièmes paramètres de variabilité de fréquence cardiaque correspondant respectivement à la pluralité de deuxièmes périodes de temps, du deuxième paramètre de variabilité de fréquence cardiaque statique comprend :
la détermination d'un deuxième paramètre de variabilité de fréquence cardiaque maximal dans les deuxièmes paramètres de variabilité de fréquence cardiaque correspondant respectivement à la pluralité de deuxièmes périodes de temps ; et
la détermination du deuxième paramètre de variabilité de fréquence cardiaque maximal en tant que deuxième paramètre de variabilité de fréquence cardiaque statique.
